# EUROPEAN PATENT SPECIFICATION

(11) **EP 4 059 598 B1**
(45) Date of publication and mention of the grant of the patent: **11.03.2026**
(21) Application number: 22172272.1
(22) Date of filing: 13.06.2019
(51) Int. Cl.: B01J 13/00, A23L 29/10

(54) **OIL-IN-WATER PICKERING EMULSION**
ÖL-IN-WASSER-BEIZEMULSION
ÉMULSION DE PICKERING HUILE DANS L'EAU

(30) Priority: 13.06.2018 WO PCT/JP2018/022612
(43) Date of publication of application: 21.09.2022
(62) Divisional of application: 19818929.2
(73) Proprietor: Mitsubishi Chemical Corporation, Chiyoda-ku Tokyo 100-8251 (JP)
(72) Inventor: Hanasaki, Minako, Chiyoda-ku, Tokyo, 100-8251 (JP); Matsuura, Tsutashi, Chiyoda-ku, Tokyo, 100-8251 (JP); Isojima, Tatsushi, Chiyoda-ku, Tokyo, 100-8251 (JP)
(74) Representative: Hoffmann Eitle

(56) References cited:
- EP-A2- 0 987 004
- US-A1- 2002 022 007
- US-A1- 2002 054 890
- US-A1- 2018 078 794
- DATABASE WPI Week 200674, Derwent World Patents Index; AN 2006-713108, XP002803502
- DATABASE WPI Week 201060, Derwent World Patents Index; AN 2010-L49661, XP002803501
- DATABASE WPI Week 201662, Derwent World Patents Index; AN 2016-38461C, XP002807139
- DATABASE WPI Week 201165, Derwent World Patents Index; AN 2011-L70383, XP002807140

## Description

### TECHNICAL FIELD

The present invention relates to an oil-in-water Pickering emulsion.

### BACKGROUND ART

In the food field, emulsification using a surfactant has been conventionally utilized. Since emulsification using a surfactant leads to thermodynamic instability, securing of long-term stability and stability against a high-temperature sterilization process requires, for example, reduction of the oil-droplet particle size of an oil-in-water (O/W) emulsion to a submicron level.

In recent years, in the food field, there are increasing needs for appetizing appearances, flavors (which stimulate the senses of taste and smell), and mouthfeel; interests in ingredients due to health consciousness; and the like. Therefore, there is a demand for development of oil-in-water emulsions having an emulsion size and a structure that are different from those of conventional emulsion compositions using surfactants.

For example, Patent Document 1 reports in Reference Example 1 that, by increasing the fat particle size of cow milk (average fat particle size, 0.4 to 1.3 µm), an increased sense of richness and a higher overall score can be achieved in sensory evaluation. Further, Patent Document 2 reports that an emulsion composition having an average emulsion particle size of 15 to 100 µm has a favorable taste. Non-patent Document 1 reports that puddings containing an emulsified oil/fat having an average particle size of 8 µm tend to give stronger senses of "sweetness" and "richness", and also "persistent aftertaste" and "deliciousness". The document thus reports that an optimal size that gives the sense of deliciousness may be about 8 µm in terms of the average particle size.

A known example of a method of stabilizing an emulsion other than emulsification using a surfactant is use of microparticles such as colloids. An emulsion stabilized by adsorption of microparticles on a liquid-liquid interface such as an oil-water interface is called "microparticle-stabilized emulsion" or "Pickering emulsion". In recent years, microparticle-stabilized emulsions (Pickering emulsions) are being actively studied.

For example, the methods of Non-patent Document 2 and Non-patent Document 3 have been disclosed in the food field.

Patent Document 3 discloses an O/W emulsion comprising fat and oil, water, water-insoluble dietary fiber, protein, and emulsifier.

Patent Document 4 is concerned with an oil-in-water emulsified oil and fat composition that does not contain an allergy labeling target substance and is excellent in emulsification stability and flavor.

Patent Documents 5, 7 and 8 describe emulsifier-free O/W or W/O Pickering emulsions containing microfine particles for use in cosmetic and dermatological preparations. Patent Document 6 relates to cosmetic and dermatologic foam formulations based on oil-in-water emulsions that are (essentially) free of conventional emulsifiers.

### PRIOR ART DOCUMENTS

### [Patent Documents]

[Patent Document 1] WO 2012/026476 A1
[Patent Document 2] WO 2015/147043 A1
[Patent Document 3] JP 2010 193811 A
[Patent Document 4] JP 2006 271279 A
[Patent Document 5] US 2002/054890 A1
[Patent Document 6] US 2018/078794 A1
[Patent Document 7] EP 0 987 004 A2
[Patent Document 8] US 2002/022007 A1

### [Non-patent Documents]

[Non-patent Document 1] Preparation Techniques for Emulsions: Case Examples, 2012, 217-223
[Non-patent Document 2] Yuan Zou et al., J. Agric. Food Chem. 2015, 63, 7405-7414
[Non-patent Document 3] Zhi-Ming Gao et al., J. Agric. Food Chem. 2014, 62, 2672-2678
[Non-patent Document 4] Oil and Fat. Vol. 65, No. 4 (2012), 94-102

### SUMMARY OF THE INVENTION

### PROBLEMS TO BE SOLVED BY THE INVENTION

However, in spite of the fact that heat resistance is required for application to food, stability upon heating is not mentioned in any of Patent Documents 1 and 2, and Non-patent Documents 2 and 3.

Further, for example, for oil-in-water emulsions using an edible oil or fat as an oil phase component, suppression of coalescence of oil droplets, suppression of creaming which leads to the coalescence, and emulsion instability which occurs as a result of interfacial breakage due to needle crystal growth of a component constituting the oil droplet have been significant issues (Non-patent Document 4). Nevertheless, the emulsion instability which occurs as a result of the needle crystal growth is not mentioned in Non-patent Document 1.

In view of this, a first object of the present invention is to provide an oil-in-water Pickering emulsion having good heat resistance and emulsion stability.

Further, because of an increase in the number of patients with food allergy who develop allergy to food, use of allergenic substances is sometimes restricted. Therefore, food ingredients used are attracting attention. Food allergy is dangerous since it may cause not only skin itching and inflammation, but also severe symptoms that may be lethal, such as anaphylactic shock. Thus, for providing oil-in-water emulsions for application to food, allergenic materials in the food ingredients used need to be replaced with other food materials depending on substances allergenic to the eaters.

For example, Non-patent Document 2 and Non-patent Document 3 disclose methods using zein contained in maize. However, zein is an ingredient of which oral ingestion should be avoided in cases where the eater has allergy to maize. For the purpose of providing a beverage having a flavor which meets the needs of consumers, Patent Document 1 discloses providing of a milk beverage which achieves an improved flavor without adjusting components of the milk beverage. However, for patients with milk allergy, use of milk-derived proteins, especially those having high allergenicity such as casein and a milk serum protein β-lactoglobulin, as food ingredients needs to be avoided.

In view of this, a second object of the present invention is to provide an oil-in-water Pickering emulsion that can be used as a substitute of an edible ingredient containing a particular allergenic substance, depending on substances allergenic to eaters.

A third object is to achieve the first object and the second object at the same time.

### MEANS FOR SOLVING THE PROBLEMS

The present inventors intensively studied to solve the above problem, and discovered that the above problem can be solved by using particular solid particles, thereby reaching the present invention. The present invention is defined as in the claims and based on the "second embodiment" described further below in the present description.

### EFFECT OF THE INVENTION

The present invention can provide an oil-in-water Pickering emulsion (hereinafter also referred to as oil-in-water emulsion composition) having good heat resistance. More specifically, the present invention can provide a Pickering emulsion whose emulsion stability can be maintained even after a high-temperature process such as sterilization, and whose change in the oil-phase particle size distribution caused by heating is small.

Since the change in the particle size distribution caused by heating is small, and since the particle size of the oil phase is appropriately large, a Pickering emulsion with good mouthfeel, appearance, tactile feeling, viscosity, stability, and the like can be provided.

Further, a Pickering emulsion having good cooling stability and/or heating stability can be provided. Thus, even when a change in the state of the oil phase component (such as coagulation, crystallization, or the like due to cooling; melting due to heating; or the like) occurs to cause a change in the surface tension of the oil phase, emulsion instability due to, for example, interfacial breakage caused by needle crystal growth of the oil phase component can be suppressed, so that the emulsion stability of the Pickering emulsion can be maintained.

Pickering emulsions provided by the present invention can be suitably used as food and beverages such as beverages and liquid food; pharmaceuticals such as liquid oral compositions; cosmetics; contrast agents; diagnostic compositions for test kits useful for POCT; medical materials; compositions for preparation of microparticles; intermediate compositions for their production; and the like.

Further, preferably, according to the present invention, depending on a substance allergenic to an eater, a Pickering emulsion free of the particular substance can be prepared to provide the Pickering emulsion as a substitute of an edible material containing the particular allergenic substance. Thus, wider options of food can be made available.

### BRIEF DESCRIPTION OF THE DRAWINGS

Fig. 1 shows a polarization micrograph (drawing-substituting photograph) of an emulsion prepared by 10-fold diluting the oil-in-water Pickering emulsion according to Example 1 with demineralized water.
Fig. 2 shows micrographs (drawing-substituting photographs) of the oil-in-water Pickering emulsions according to (A) Example 4, (B) Example 5, and (C) Example 6.
Fig. 3 shows micrographs (drawing-substituting photographs) of the oil-in-water Pickering emulsions according to (A) Example 7 and (B) Example 8.
Fig. 4 shows a micrograph (drawing-substituting photograph) of the oil-in-water Pickering emulsion according to Example 9.
Fig. 5 shows micrographs (drawing-substituting photographs) of the oil-in-water Pickering emulsion according to Example 4. The photograph of (A) is a micrograph obtained by observation in the open-Nicol configuration, and the photograph of (B) is a micrograph obtained by observation in the cross-Nicol configuration (drawing-substituting photographs).
Fig. 6 shows a micrograph (drawing-substituting photograph) of the oil-in-water Pickering emulsion according to Example 18.

### MODE FOR CARRYING OUT THE INVENTION

Embodiments of the present description and invention are described below in detail. The descriptions of the constituents described below are merely examples of embodiments (representative examples) of the present invention, and the present invention is not limited thereto.

The first embodiment of the present description is described below.

The oil-in-water Pickering emulsion (oil-in-water emulsion composition) of the first embodiment is an oil-in-water Pickering emulsion comprising a solid particle, a nonionic amphiphilic substance, an oil phase component, and an aqueous phase component, and the solid particle is an organic substance.

In the present description, the oil-in-water Pickering emulsion includes not only the so-called O/W oil-in-water Pickering emulsions, wherein an aqueous phase is present as the continuous phase, but also multiphase emulsions such as W/O/W Pickering emulsions.

### (Solid Particle)

In the present embodiment, the solid particle is an organic substance. The solid particle is not limited, and an arbitrary organic substance may be used as the solid particle as long as it is insoluble in the aqueous phase component and the oil phase component used, and as long as the aqueous phase and/or the oil phase can be stirred even after the solid particle is added to the aqueous phase component and/or the oil phase component. A single kind of solid particles may be used, or two or more kinds of solid particles arbitrarily selected may be used in combination.

The "solid" means a state where the particles have no fluidity in the temperature history from preparation of the composition to consumption. The solid particles before the dispersion in the aqueous phase or the oil phase may be in a form of powder, paste, or pellet.

Examples of the organic substance include polysaccharides such as chitin, chitosan, cellulose, microcrystalline cellulose, hydroxypropyl methylcellulose, hydroxycellulose, methylcellulose, fermented cellulose, sodium carboxymethylcellulose, gellan gum, native gellan gum, xanthan gum, carrageenan, dextrin, indigestible dextrin, soybean polysaccharides, pectin, alginic acid, propylene glycol alginate, tamarind seed gum, tara gum, karaya gum, guar gum, locust bean gum, tragacanth gum, ghatti gum, pullulan, gum arabic, agar, furcellaran, inulin, and konjac mannan; polymers such as polylactic acid, polyvinyl alcohol, and polyethylene glycol; organic pigments; oligomers; Janus particles; starch; processed starch products; cyclodextrin; animal proteins such as whey and casein; plant proteins such as soybean protein and zein; microorganism-derived proteins such as hydrophobin; enzymes; protein degradation products; peptides; microorganisms; spores; cells; plant extracts such as flavonoids; ground food products such as ground protein gel products and cereal powders; and complexes and derivatives thereof. The organic substance may be either a synthetic product or a natural product. In particular, in cases of a polysaccharide or a polymer, the substance may be linear (cellulose), branched (glucomannan and the like), side-chained (galactomannans), or spherical (gum arabic and soybean polysaccharides). The substance may be an acidic polysaccharide, a neutral polysaccharide, or a basic polysaccharide.

The raw material from which the starch is derived is not limited. Representative examples of the raw material include potato, waxy potato, wheat, maize, waxy maize, high-amylose maize, sweet potato, rice, waxy rice, cassava, kudzu, dog-tooth violet, mung bean, sago palm, bracken, and *Cardiocrinum cordatum* var. *glehnii* (ooubayuri) .

Examples of the processed starch products include processed starches and chemically modified starches prepared by subjecting starch to various wet or dry (enzymatic, physical, or chemical) processes to improve properties or to impart functionalities to the starch. Specific examples of the processed starch products include enzyme-treated starch, sodium starch glycolate, sodium starch phosphate, acetylated distarch adipate, acetylated distarch phosphate, acetylated oxidized starch, hydroxypropyl distarch phosphate, phosphated distarch phosphate, monostarch phosphate, distarch phosphate, starch acetate, hydroxypropyl starch, starch sodium octenyl succinate, oxidized starch, acid-treated starch, gelatinized starch, dried starch, heat-treated starch, heat-moisture-treated starch, oil/fat-processed starch, granulated starch, and oil-absorbing starch.

The starch content with respect to the total weight of the solid particles is preferably less than 50% by weight, more preferably not more than 40% by weight, still more preferably not more than 25% by weight, especially preferably not more than 1% by weight. The solid particles most preferably do not contain starch at all. In cases where the starch content in the solid particles exceeds this range, the viscosity of the whole composition cannot be easily kept constant during heat sterilization. Further, retrogradation and crystallization of once-gelatinized starch may precipitate and thus be separated, leading to insufficient thermal stability.

In cases where the Pickering emulsion of the present embodiment is used as a composition for oral ingestion, for example, in cases where it is used in the food field, the solid particles may be an edible organic substance, and may be either a food additive or a food ingredient. Preferred examples of the edible organic substance include a protein. The protein may be a natural protein, or may be a synthetic protein prepared by the genetic recombination technique or the like. The protein may also be a modified protein; a protein partially degraded by acid treatment, alkali treatment, enzyme treatment, or the like; or a peptide.

The isoelectric point of the protein used is not limited. Regarding the lower limit of the isoelectric point of the protein, which is a major component constituting the solid particles, the pH is usually not less than 2, preferably not less than 3, more preferably not less than 4. Regarding the upper limit, the pH is usually not more than 12, preferably not more than 11, more preferably not more than 10, especially preferably not more than 9, most preferably not more than 8. In cases where the isoelectric point is too low or too high, the protein itself has an increased affinity to the aqueous phase component or oil phase component. This leads to difficulty in maintaining appropriate wettability.

The protein is preferably a hydrophobic protein. In cases where a hydrophobic protein is used, the later-mentioned amphiphilic substance adsorbs to the hydrophobic protein, that is, the solid particles, through a hydrophobic site of the amphiphilic substance, thereby modifying the surface properties of the solid particles. In this process, by changing the type of the amphiphilic substance, the amount of the amphiphilic substance added, and/or the extent of complexation of the amphiphilic substance, the wettability of the solid particle can be controlled in accordance with the oil phase component used, so that a Pickering emulsion can be more easily formed. In contrast, in cases where a hydrophilic protein is used, because electrostatic interaction and the like works between it and the amphiphilic substance, the materials used, the service conditions (such as the pH and the salt concentration), and the like, are limited in dissolving the protein in water or changing the surface wettability of the solid particle, therefore the protein has a drawback in its applicability.

The protein may be subjected to UV irradiation; physical treatment by heat or pressure; and/or chemical treatment using an acid, alkali, denaturant (such as urea, guanidine hydrochloride, an organic solvent such as alcohol, or a surfactant), enzyme, oxidizing agent, reducing agent, chelating agent, or the like. By performing such treatments, the protein can be physically and/or chemically modified (for example, denatured) to thereby control the wettability of the protein itself or that of a particle (a protein aggregate, or a complex containing the protein) itself formed by the protein. Thus, the protein having an appropriately-controlled wettability tends to be present on the interface (interface between the oil phase and the aqueous phase) to be stabilized, which enables formation of a stable Pickering emulsion. With the treatment such as heating, pressurization, and/or UV irradiation, a sterilization effect which prevents rotting of the materials themselves can also be expected.

One of these treatments may be carried out alone, or arbitrarily selected two or more treatments may be carried out simultaneously or with an interval. For example, a denaturant is added to a solution containing the protein, and then heat is applied to the resulting mixture. By this, denaturation treatment with the denaturant and denaturation treatment with the heat are carried out simultaneously. The method of the denaturation treatment may be selected taking into account the type of the protein to be denatured, the degree of denaturation required, and the like. For example, in cases where heat treatment is carried out, the treatment may be either dry heating or wet heating. The apparatus used is not limited. For example, in the case of dry heating, a roasting apparatus, a hot-air heating apparatus, a microwave heating apparatus, or the like may be used. In the case of wet heating, a humidifying/heating apparatus, a steaming apparatus, a steam-heating apparatus, or the like may be used. The heating temperature is usually not less than 30°C, preferably not less than 40°C, more preferably not less than 50°C, still more preferably not less than 60°C, still more preferably not less than 70°C, still more preferably not less than 80°C. The upper-limit temperature is not limited as long as the protein does not undergo complete degradation or evaporation, that is, as long as the temperature is less than 200°C. The length of time of the heat treatment may be arbitrary. The length of time is usually not less than 10 seconds, preferably not less than 30 seconds, more preferably not less than 1 minute, still more preferably not less than 5 minutes, still more preferably not less than 10 minutes, especially preferably not less than 15 minutes, most preferably not less than 30 minutes.

The hydrophobic protein means a protein having a high content of hydrophobic amino acids in the constituent amino acids. Because of the inclusion of a large amount of hydrophobic amino acids, the water solubility of the protein decreases, and hence a hydrophobic protein is formed. Examples of the hydrophobic amino acids include leucine, isoleucine, valine, phenylalanine, proline, glutamine, and asparagine.

The hydrophobic protein can be evaluated also by measurement of the contact angle of water. The contact angle is usually not less than 5°, preferably not less than 10°, more preferably not less than 15°, still more preferably not less than 20°, still more preferably not less than 40°, especially preferably not less than 50°, most preferably not less than 65°. Although there is no upper limit of the contact angle, from the viewpoint of ease of handling during the production, for example, for dispersion in the aqueous phase, the contact angle is usually less than 180°, preferably not more than 150°, more preferably not more than 130°, still more preferably not more than 110°, especially preferably not more than 90°, most preferably not more than 80°.

The measurement method for the contact angle is as follows. The solid particles are prepared into a tablet, water is dropped thereon by its own weight, and the contact angle is then measured using a contact angle measuring device at normal temperature. More specifically, the contact angle may be measured by the measurement method described in Examples.

Examples of the hydrophobic protein include glutelin, prolamin, and globulin. Glutelin or prolamin is preferred since these have high hydrophobicity. Examples of the prolamin include zein, gliadin, hordein, and kafirin.

The hydrophobic protein may be an animal protein derived from an animal, may be a plant protein derived from a plant, or may be a protein derived from a fungus. Patients with milk allergy need to avoid milk-derived proteins, especially casein and a milk serum protein β-lactoglobulin, which are highly allergenic, as food ingredients. Therefore, plant proteins are preferred. Also, from the viewpoint of providing food that meets the needs of religious restrictions and vegetarians, use of a plant protein derived from a plant is preferred.

Examples of the plant include various cereals (major cereals, millets, legumes, and pseudo-cereals) such as maize (corn), wheat, barley, oat (karasumugi), rye, rice (rice seed), sorghum (morokosi), sorghum, Japanese millet, foxtail millet, proso millet, buckwheat, amaranthus, and quinoa, and beans such as pea, soybean, and mung bean.

In a preferred mode, from the viewpoint of allergenicity, examples of the plant include those other than wheat, buckwheat, and peanut, which are included in the 7 specified raw materials for which labeling is mandatory among the items subject to allergen labeling for processed food under the Food Sanitation Law, and other than cashew nut, walnut, sesame, soybean, and yam, which are included in the 20 items additional to the specified raw materials and for which labeling is recommended. Preferred specific examples of the plant include maize (corn), barley, oat (karasumugi), oat, rye, rice (rice seed), sorghum (morokosi), sorghum, Japanese millet, foxtail millet, proso millet, amaranthus, quinoa, pea, and mung bean. Unlike beans, which contain globulin as a major protein, gramineous plants contain glutelin and prolamin as major proteins. Thus, proteins derived from gramineous plants are more preferred because of their higher hydrophobicity. Accordingly, the plant is more preferably maize (corn), barley, oat (karasumugi), oat, rye, rice (rice seed), sorghum (morokosi), sorghum, Japanese millet, foxtail millet, or proso millet, still more preferably maize (corn) or rice (rice seed), most preferably rice (rice seed).

In some cases, the taste, color, or smell of the solid particles themselves affects the use for a food or the like to be orally ingested. Thus, from the viewpoint of flavor, protein derived from rice is more preferably used than protein derived from maize (corn), which has a characteristic smell.

In cases where the solid particles have a dark and dense color, there may be a limitation in the use thereof. For example, for achievement of a desired taste, color, and smell of food, colored solid particles may require addition of a larger amount of seasoning materials, coloring agents, or flavorings, than colorless solid particles. As a result, the number of production steps and the amount of additives tend to increase, so that the production may be more laborious and costly.

Thus, the solid particles used in the present embodiment have an L value of usually not less than 31, preferably not less than 40, more preferably not less than 50, still more preferably not less than 62. Although the upper limit is not limited, the L value is usually not more than 100. In cases where the solid particles have such a large L value, the oil-in-water Pickering emulsion has a favorable appearance.

On the other hand, examples of solid particles having an L value smaller than the above-described range include components derived from roasted coffee beans having a dark and deep color. Such components derived from roasted coffee beans tend to have a smaller L value as the degree of roasting increases, so that the appearance of the oil-in-water Pickering emulsion may be adversely affected. Regarding the relationship between the degree of roasting and the L value, for example, in the robusta species produced in Indonesia, unroasted beans have an L value of 57; light-roasted beans have an L value of 32; medium-roasted beans have an L value of 20; and deep-roasted beans have an L value of 16. In the arabica species produced in Colombia, unroasted beans have an L value of 55, light-roasted beans have an L value of 32, medium-roasted beans have an L value of 20, and deep-roasted beans have an L value of 16.

The L value of the solid particles may be measured using a colorimeter. The L value represents brightness of color, and is expressed as a value of 0 to 100. An L value of 100 represents the brightest state (complete white), and an L value of 0 represents the darkest state (complete black). The measurement using a colorimeter may be carried out by a known measurement method.

One example of an index for evaluation of the nutritive value of a protein is the amino acid score. Rice has an amino acid score of 61, which is higher than those of other major cereals. Regarding the other major cereals, for example, wheat (bread flour) has an amino acid score of 36, and maize (corn grits) has an amino acid score of 31. Thus, rice has a good balance of amino acids, and is excellent from the nutritional point of view. Therefore, in the present embodiment, rice-derived protein is preferred among hydrophobic proteins. Preferred examples of the rice-derived protein include glutelin (oryzenin), prolamin, globulin, and albumin. Glutelin and/or prolamin is/are preferred since they have high hydrophobicity. Glutelin and/or protamine is/are preferred also because some eaters are allergic to globulin and albumin, which are present in small amounts in rice.

In rice, prolamin and glutelin are reserve proteins accumulated in protein body I and protein body II, respectively. Therefore, they can be obtained by separating globulin and albumin from rice by, for example, a treatment method such as "J. Agric. Food Chem. 2000, 48, 3124-3129"; an extraction/purification method using water, an acid, an alkali, an organic solvent, or a salt, such as "JP 2007-68454 A" or "JP 5819981 B"; a globulin/albumin-specific degradation treatment using an enzyme; or combination of these treatment methods.

The amino acid score is a percentage value representing the ratio of the most insufficient amino acid based on comparison of the amounts of the essential amino acids per protein in a food with those in the amino acid pattern proposed by the joint committee of the Food and Agriculture Organization (FAO), World Health Organization (WHO), and United Nations University (UNU) in 1985, wherein a value of 100 indicates that all amino acids are sufficient. Among the amino acid patterns for different age groups, the pattern for ages 2 to 5 years, which is commonly employed, was used for performing the calculation in the present invention.

The shape of the solid particles is not limited, and examples of the shape include a spherical shape, a rod-like shape, a string-like shape, a gelatinous shape, a net-like shape, a porous shape, a needle-like shape, and a flaky shape. In cases where the solid particles have a gelatinous shape, it may be shrunken or swollen.

The solid particles may be formed by a single component, or may be formed by a mixture composed of a plurality of different kinds of components.

The solid particles may or may not be forming an aggregate or an associated body. In cases where the solid particles are forming an aggregate or an associated body, and are composed of a macromolecule, the solid particles preferably have an entangled structure, and/or a cross-linked structure formed by, for example, hydrogen bond, ionic bond, or intermolecular force between them.

The solid particles may contain an effective component therein and/or on the surface layer.

The primary particle size of the solid particles is not limited, and may be appropriately selected depending on, for example, the particle size of the oil phase, or the type of the organic substance constituting the solid particles. The primary particle size is usually not less than 0.001 µm, preferably not less than 0.01 µm, preferably not less than 0.05 µm, still more preferably not less than 0.1 µm, especially preferably not less than 0.5 µm, and is usually not more than 50 µm, preferably not more than 5 µm, more preferably not more than 1 µm, especially preferably not more than 0.9 µm.

The primary particle size of the solid particles means, for example, the average particle size of particles observable in an image, which image is obtained by performing scanning electron microscopic (SEM) measurement and magnifying the resulting particle image. The number of particles observed may be not less than 5, may be not less than 20, may be not less than 40, may be not less than 100, or may be not less than 200. As the primary particle size of the solid particles, a catalog value may be used.

The average particle size of the solid particles is not limited, and may be appropriately selected depending on, for example, the particle size of the oil phase, or the type of the organic substance constituting the solid particles. The average particle size of the solid particles dispersed in a diluted state in the liquid is usually not less than 0.01 µm, preferably not less than 0.05 µm, more preferably not less than 0.1 µm, especially preferably not less than 0.5 µm, and is usually not more than 1000 µm, preferably not more than 500 µm, more preferably not more than 250 µm, more preferably not more than 100 µm, more preferably not more than 50 µm, more preferably not more than 5 µm, more preferably not more than 3 µm, still more preferably not more than 1 µm, especially preferably not more than 0.9 µm. The diluted state herein means an arbitrary concentration which can be measured, for example, by the flow method using a laser diffraction/scattering particle size distribution measuring apparatus. The concentration at which the measurement is carried out is usually not more than 20% by weight, preferably not more than 5% by weight, more preferably not more than 1% by weight, still more preferably not more than 0.1% by weight, especially preferably not more than 0.02% by weight.

Regarding the size of the solid particles in the liquid, for example, a laser diffraction/scattering particle size distribution measuring apparatus may be used to measure the particle size distribution, average particle size, and median size of the solid particles in a powder state or in a state where they are dispersed in a liquid.

In cases where the measurement using a laser diffraction/scattering particle size distribution measuring apparatus is difficult because of, for example, insufficient intensity of the diffracted/scattered light, measurement by the dynamic light scattering method may be carried out to measure the particle size distribution, average particle size, and median size of the solid particles in a state where they are dispersed in a liquid. The measurement result by the dynamic light scattering method may be analyzed by, for example, the cumulant method. In cases where the measurement is possible either using a laser diffraction/scattering particle size distribution measuring apparatus or by the dynamic light scattering method, it is preferred to carry out the measurement using a laser diffraction/scattering particle size distribution measuring apparatus.

From the viewpoint of dispersion and particle size control of the solid particles constituting the oil-in-water emulsion composition of the present embodiment, the solid particles may be additionally subjected to crushing treatment, pulverization treatment, or dispersion treatment. These treatment methods are not limited, and either treatment by a dry method or treatment by a wet method may be carried out. These may be carried out in combination to perform crushing, pulverization, and/or dispersion treatment(s) in a stepwise manner.

Examples of the wet treatment method include use of a high-pressure homogenizer, a homogenizer, a jet mill, a vibration mill, a tumbling mill, a high-pressure fluid impact mill, a paint shaker, a bead mill, a ball mill, a disk mill, a homomixer, or the like, and examples of the dry treatment method include use of a pin mill, a jet mill, a ball mill, a hammer mill, a roller mill, a cutter mill, an impact shearing mill, or the like. Use of a high-pressure homogenizer, a bead mill, a cutter mill, or a hammer mill is preferred.

In cases where beads are used in the wet treatment, beads having a diameter of about 0.05 to 5 mm are preferably used. The material of the beads is not limited, and the beads may be, for example, glass beads, special glass beads, alumina beads, zirconia-silica ceramic beads, zirconia beads, silicon nitride beads, or steel.

The temperature during the treatment is usually not less than -196°C, preferably not less than -80°C, more preferably not less than -40°C, still more preferably not less than -20°C, still more preferably not less than 0°C, especially preferably not less than room temperature. Regarding the upper limit of the temperature during the treatment, the temperature is usually not more than 100°C, preferably not more than 90°C, more preferably not more than 80°C.

The treatment time is usually not less than 30 seconds, preferably not less than 1 minute, more preferably not less than 1 minute and 30 seconds, still more preferably not less than 2 minutes, still more preferably not less than 30 minutes, especially preferably not less than 1 hour, most preferably not less than 2 hours. The treatment time is usually not more than 10 hours, preferably not more than 8 hours, more preferably not more than 7 hours, still more preferably not more than 6 hours. In cases where the treatment time is too short, control of the particle size tends to be difficult, while in cases where the treatment time is too long, the productivity tends to be low.

For production of the solid particles as a component of the oil-in-water emulsion composition of the present embodiment, the crushed particles obtained by the above production method may be subjected to classification treatment based on the particle size.

Regarding the classification conditions, the mesh size is usually not more than 150 µm, preferably not more than 106 µm, more preferably not more than 53 µm, still more preferably not more than 45 µm, still more preferably not more than 38 µm, especially preferably not more than 20 µm.

The apparatus used for the classification treatment is not limited. For example, in cases of dry sieving, a trommel sieve, an agitating sieve, a rotary sieve, a vibrating sieve, or the like may be used. In cases of dry airflow classification, a gravity classification apparatus, an inertial-force classification apparatus, a centrifugal classification apparatus (classifier, cyclone, or the like), or the like may be used. In cases of wet classification, a mechanical wet classification apparatus, a hydraulic classification apparatus, a sedimentation classification apparatus, a centrifugal wet classification apparatus, or the like may be used.

The size of the solid particles which is present on the aqueous phase-oil phase interface in the oil-in-water Pickering emulsion according to the present embodiment is not limited. The size of the solid particles is usually not less than 0.01 µm, preferably not less than 0.05 µm, more preferably not less than 0.1 µm, especially preferably not less than 0.5 µm, and is usually not more than 500 µm, preferably not more than 250 µm, more preferably not more than 100 µm, more preferably not more than 50 µm, more preferably not more than 20 µm, more preferably not more than 5 µm, more preferably not more than 3 µm, still more preferably not more than 1 µm, especially preferably not more than 0.9 µm.

The particle size of the solid particles present on the aqueous phase-oil phase interface means, for example, the average particle size of particles observable in an image, which image is obtained by performing light microscopic or scanning electron microscopic (SEM) measurement and magnifying the particle image obtained. It is preferred to use an operable electron microscope for the observation. The number of particles observed may be not less than 5, may be not less than 40, may be not less than 100, or may be not less than 200.

In cases where the measurement using a laser diffraction/scattering particle size distribution measuring apparatus is difficult because of, for example, insufficient intensity of the diffracted/scattered light, measurement by the dynamic light scattering method may be carried out to measure the particle size distribution, average particle size, and median size of the solid particles in a state where they are dispersed in a liquid. The measurement result by the dynamic light scattering method may be analyzed by, for example, the cumulant method.

The content of the solid particles in the oil-in-water Pickering emulsion according to the present embodiment is not limited as long as in a range where the solid particles can be normally contained in the Pickering emulsion. The content is usually not less than 0.01% by weight, preferably not less than 0.05% by weight, more preferably not less than 0.1% by weight, most preferably not less than 0.5% by weight, and is usually not more than 50% by weight, preferably not more than 40% by weight, more preferably not more than 30% by weight, especially preferably not more than 20% by weight, most preferably not more than 15% by weight, with respect to the total amount of the composition.

### (Nonionic Amphiphilic Substance)

In the present embodiment, use of a nonionic amphiphilic substance enables control of the surface wettability of various solid particles. Since nonionic amphiphilic substances are less likely to be affected by the pH and salts than ionic amphiphilic substances, applicability of the nonionic amphiphilic substances is less likely to be limited due to the solid particles, the oil phase component, the aqueous phase component, or other components.

The amphiphilic substance is usually a substance having an amphiphilic structure in the molecule, preferably a surface-active substance. Examples of the amphiphilic substance include amphiphilic polymers, proteins, phospholipids, and surfactants containing a hydrophobic segment and a hydrophilic segment. The nonionic amphiphilic substance in the present embodiment is preferably a low molecular weight surfactant, and its molecular weight is preferably not more than 5000, more preferably not more than 3000, most preferably not more than 2000. The lower the molecular weight of the low molecular weight surfactant, the larger the number of moles thereof per weight, which is preferred since a larger number of molecules can contribute to the reaction with the solid particles. The lower limit of the molecular weight of the low molecular weight surfactant is not limited. Since a hydrophilic portion and a lipophilic portion are contained in the molecular structure, the molecular weight is usually not less than 200.

In cases where the low molecular weight surfactant contains an alkyl group as a lipophilic group, the alkyl group may be either a linear alkyl group or a branched alkyl group. The alkyl group is preferably a linear alkyl group. Regarding the chain length of the alkyl group, the alkyl group has usually not less than 8, preferably not less than 10, more preferably not less than 12, still more preferably not less than 14, most preferably not less than 16 carbon atoms. Although there is no upper limit of the number of carbon atoms, it is usually not more than 24, preferably not more than 22.

The lipophilic group of the low molecular weight surfactant may also be an alkenyl group or an alkynyl group, which has an unsaturated bond. The presence or absence of branching, chain length, and preferred modes of these groups are the same as those in the case of an alkyl group. In the present embodiment, the lipophilic group is preferably an alkyl group, which has no unsaturated bond.

In the present embodiment, the low molecular weight surfactant does not contain a macromolecule such as protein, polysaccharide, or synthetic polymer. The nonionic amphiphilic substance may be in any form such as powder, solid, liquid, or paste. A single kind of nonionic amphiphilic substance may be used, or two or more kinds of different amphiphilic substances may be used in combination.

The HLB of the nonionic amphiphilic substance is not limited, and preferably not less than 3. In cases where the HLB is within this range, the substance can be easily used by dispersion and/or dissolution in the aqueous phase component, which is advantageous. The HLB of the nonionic amphiphilic substance is usually not less than 0, preferably not less than 3, more preferably not less than 5, still more preferably not less than 7, especially preferably not less than 10, most preferably not less than 12. The HLB is preferably not more than 20, more preferably not more than 19, still more preferably not more than 18.

The HLB value usually represents the balance between the hydrophilicity and the hydrophobicity, and used in the field of surfactants. A commonly used calculation equation such as the Griffin method, Davis method, Kawakami method, or a conceptual organic substance chart may be used therefor. An HLB value described in a catalog or the like may also be used.

The nonionic amphiphilic substance is not limited, and may be a fatty acid ester such as a sucrose fatty acid ester, a glycerin fatty acid ester, a polyglycerin fatty acid ester, a sorbitan fatty acid ester, or a propylene glycol fatty acid ester; or derivatives thereof. Among these, from the viewpoint of bacteriostatic properties against heat-resistant bacteria in cases of use for oral ingestion, the nonionic amphiphilic substance is preferably a sucrose fatty acid ester, a polyglycerin fatty acid ester, or the like, especially preferably a sucrose fatty acid ester.

In cases where the nonionic amphiphilic substance is a polyglycerin fatty acid ester, from the viewpoint of the bacteriostatic effect against heat-resistant bacteria, the fatty acid group preferably has 14 to 22 carbon atoms, more preferably 16 to 18 carbon atoms. From the viewpoint of the bacteriostatic effect against heat-resistant bacteria, the monoester content in the polyglycerin fatty acid ester is usually not less than 5% by weight, preferably not less than 10% by weight, more preferably not less than 20% by weight, more preferably not less than 50% by weight, more preferably not less than 60% by mass, still more preferably not less than 70% by mass, and usually not more than 100% by weight. From the viewpoint of the bacteriostatic effect against heat-resistant bacteria, the average degree of polymerization of the polyglycerin in the polyglycerin fatty acid ester is preferably 2 to 10, more preferably 2 to 5, most preferably 2 to 3.

In cases where the nonionic amphiphilic substance is a sucrose fatty acid ester, from the viewpoint of the bacteriostatic effect against heat-resistant bacteria, the fatty acid group more preferably has 14 to 22 carbon atoms, still more preferably 16 to 18 carbon atoms. The sucrose fatty acid ester may be a monoester, or may be a polyester such as a diester or a triester. The monoester content in the sucrose fatty acid ester is usually not less than 20% by weight, preferably not less than 30% by weight, more preferably not less than 40% by weight. From the viewpoint of the bacteriostatic effect against heat-resistant bacteria, the monoester content is still more preferably not less than 50% by weight, most preferably not less than 70% by weight. The monoester content is usually not more than 100% by weight, preferably not more than 99% by weight, more preferably not more than 95% by weight, especially preferably not more than 90% by weight, most preferably not more than 80% by weight.

Examples of commercially available products of the sucrose fatty acid ester include "RYOTO Sugar Ester S-1670", "RYOTO Sugar Ester S-1570", "RYOTO Sugar Ester S-1170", "RYOTO Sugar Ester S-970", "RYOTO Sugar Ester S-570", "RYOTO Sugar Ester P-1670", "RYOTO Sugar Ester P-1570", "RYOTO Sugar Ester M-1695", "RYOTO Sugar Ester O-1570", "RYOTO Sugar Ester L-1695", "RYOTO Sugar Ester LWA-1570", and "RYOTO Monoester-P" (all of which are manufactured by Mitsubishi-Chemical Foods Corporation; trade names); and "DK Ester SS", "DK Ester F-160", "DK Ester F-140", and "DK Ester F-110" (all of which are manufactured by DKS Co., Ltd.; trade names).

Examples of the reaction or interaction between the amphiphilic substance and the solid particles include hydrophobic interaction, intermolecular force interaction, hydrogen bonding, and antigen-antibody reaction. From the viewpoint of inhibition of electrostatic interaction by salts, hydrophobic interaction and/or hydrogen bonding is/are preferred. In cases where the solid particles are hydrophobic protein, hydrophobic interaction is preferred.

The method of analysis of the amphiphilic substance contained in the oil-in-water Pickering emulsion is not limited. For example, the analysis may be carried out by the following procedures (1) to (3).
(1) The oil-in-water Pickering emulsion is centrifuged, and each of the resulting supernatant and precipitate (such as solid particles to which the amphiphilic substance is adsorbed) is collected and analyzed.
(2) From the precipitate obtained in (1), the amphiphilic substance is desorbed by various methods (for example, salt addition, pH adjustment, or washing with a desired solvent such as ethanol), to obtain an extract of the amphiphilic substance.
(3) The supernatant obtained in (1) and the extract of the amphiphilic substance obtained in (2) are subjected to identification by a method such as GPC (see, for example, JP 8-269075 A), LC/MS, LC/MS/MS (see, for example, JP 2014-122213 A), GC/MS, GC/MS/MS, or NMR.

The content of the nonionic amphiphilic substance in the oil-in-water Pickering emulsion according to the present embodiment is not limited as long as in a range where the nonionic amphiphilic substance can be contained in the Pickering emulsion. The content is usually not less than 0.00001% by weight, preferably not less than 0.00005% by weight, more preferably not less than 0.0001% by weight, most preferably not less than 0.001% by weight, and is usually not more than 5% by weight, preferably less than 1% by weight, more preferably not more than 0.5% by weight, still more preferably not more than 0.1% by weight, especially preferably not more than 0.05% by weight, most preferably not more than 0.01% by weight, with respect to the total amount of the Pickering emulsion.

The weight of the nonionic amphiphilic substance with respect to the weight of the solid particles (nonionic amphiphilic substance/solid particles) is usually not less than 0.00001, preferably not less than 0.00005, more preferably not less than 0.0001, still more preferably not less than 0.0005, especially preferably not less than 0.001, most preferably not less than 0.0025, and is usually not more than 5, preferably less than 1, more preferably not more than 0.5, still more preferably not more than 0.1, still more preferably not more than 0.05, especially preferably not more than 0.01, most preferably not more than 0.005.

The concentration of the nonionic amphiphilic substance in the oil-in-water Pickering emulsion in the present embodiment is more preferably not more than the critical micelle concentration. In cases where the concentration of the nonionic amphiphilic substance is not more than the critical micelle concentration, the nonionic amphiphilic substance is capable of binding or adsorbing to the solid particles to form a single layer without forming micelles. Thus, the surface properties of the solid particles can be efficiently modified, and as a result, the amount of the nonionic amphiphilic substance added can be reduced.

### (Oil Phase Component)

In the present embodiment, the oil phase component is not limited as long as it can be used in a Pickering emulsion. Examples of the oil phase component include higher unsaturated fatty acid hydrocarbons; higher unsaturated fatty acids; animal-derived and plant-derived oils and fats; isoprenoids including squalene and tocopherol; higher alcohols; synthetic ester oils; glycol higher fatty acid esters; saturated fatty acids, and unsaturated fatty acids.

The oil phase component preferably contains a component which can be used for food (hereinafter referred to as "edible oil or fat"). Any edible oil or fat may be used. Examples of the edible oil or fat include oils and fats having physiological functions, liposoluble pigments, and antioxidants.

Examples of the edible oil or fat include plant oils and fats, such as rapeseed oil, rice oil, soybean oil, corn oil, safflower oil, sunflower oil, cottonseed oil, sesame oil, olive oil, palm oil, palm kernel oil, coconut oil, linseed oil, macadamia nut oil, camellia seed oil, tea seed oil, rice bran oil, and cocoa butter; animal oils and fats, such as milk fat, beef tallow, lard, chicken fat, mutton tallow, and fish oil; hydrogenated or processed oils and fats produced from liquid or solid products of these plant or animal oils and fats through oil or fat processing by purification, deodorization, separation, hydrogenation, or transesterification, such as hydrogenated palm oil and hydrogenated palm kernel oil; and liquid oils and solid fats obtained by further separation of these oils and fats. One of these, or two or more of these may be used. Oils and fats having physiological functions may also be used. Specific examples thereof include docosahexaenoic acid (DHA), eicosapentaenoic acid (EPA), arachidonic acid, α-linolenic acid, γ-linolenic acid, and medium-chain fatty acid triglyceride (MCT). These oils and fats may be used individually or as a mixture.

Liposoluble pigments and antioxidants may also be used. Specific examples the pigments include carotenoid pigments such as annatto pigment, β-carotene, paprika pigment, carrot carotene, and *Dunaliella* carotene; *Monascus* pigment; chlorophyll; turmeric pigments such as curcumin (curcuminoid); and edible tar pigments.

Specific examples of the antioxidants include plant extracts such as rosemary extracts, tea extracts, raw coffee bean extracts, grape seed extracts, and myrica extracts; tocopherol, tocotrienol; ascorbyl palmitate; dibutylhydroxytoluene; and butylhydroxyanisole.

The oil phase component is especially preferably a fat which is solid at normal temperature, from the viewpoint of taste. The fat which is solid at normal temperature is a solid fat that is present in a solid state at normal temperature (25°C), and examples thereof include beef tallow, lard, palm stearin, palm medium melting point fraction, hydrogenated coconut oil, hydrogenated palm kernel oil, hydrogenated rapeseed oil, hydrogenated castor oil, hydrogenated soybean oil, hydrogenated beef tallow oil, and hydrogenated fish oil. A plant oil or fat, a hydrogenated or processed oil or fat thereof, or the like is more preferably used.

More preferred examples of the oil phase component include palm oil; palm stearin; palm kernel oil; coconut oil; cocoa butter; milk fat; beef tallow; lard; chicken fat; mutton tallow, hydrogenated oils and fats of a plant or animal oil or fat, such as hydrogenated coconut oil and hydrogenated palm kernel oil; solid fats obtained by separation of a hydrogenated or processed oil or fat of a plant or animal oil or fat; and medium-chain fatty acid triglyceride (MCT). Still more preferred examples of the oil phase component include palm kernel oil, coconut oil, milk fat, hydrogenated coconut oil, hydrogenated palm kernel oil, and medium-chain fatty acid triglyceride (MCT). Most preferred examples of the oil phase component include palm kernel oil, coconut oil, hydrogenated coconut oil, and hydrogenated palm kernel oil.

These oils and fats may be used individually or as a mixture.

In particular, from the viewpoint of achieving a better taste, the ratio of fatty acids other than saturated fatty acids, that is, the ratio of unsaturated fatty acids including trans fatty acids, in the total fatty acids bound to the major component triglyceride molecules in the edible oil or fat is preferably not more than 50% by mass, more preferably not more than 30% by mass, still more preferably not more than 20% by mass, especially preferably not more than 10% by mass, most preferably not more than 5% by mass.

Further, the ratio of fatty acids containing not more than 12 carbon atoms in the total fatty acids bound to the triglyceride molecules in the edible oil or fat is preferably not less than 1% by mass, more preferably not less than 3% by mass, still more preferably not less than 5% by mass, especially preferably not less than 10% by mass, most preferably not less than 30% by mass.

From the viewpoint of prevention of an oxidized flavor upon heating, and achievement of a favorable flavor, the edible oil or fat has an iodine value of usually not more than 60.0, preferably not more than 50.0, more preferably not more than 30.0, still more preferably not more than 20.0, especially preferably not more than 10.0, most preferably not more than 5.0.

From the viewpoint of preparation of a composition having a good flavor, the edible oil or fat has an SFC (solid fat content), at 10°C, of usually not less than 0% by mass, preferably not less than 20% by mass, more preferably not less than 30% by mass, still more preferably not less than 40% by mass, most preferably not less than 50% by mass.

Here, the measurement of the solid fat content (SFC) is commonly carried out by an ordinary pulse NMR method. A similar result can be obtained by use of the solid fat index (SFI) obtained by thermal analysis.

From the viewpoint of preparing a composition having a good flavor, the edible oil or fat has a slip melting point of usually not less than -20°C, preferably not less than -10°C, more preferably not less than 10°C, still more preferably not less than 15°C, especially preferably not less than 20°C, most preferably not less than 25°C. Regarding the upper limit, from the viewpoint of obtaining a good emulsion stability, the slip melting point is preferably not more than 70°C, more preferably not more than 60°C, still more preferably not more than 50°C, most preferably not more than 45°C.

In the present embodiment, the average particle size of the oil phase is preferably not less than 2.2 µm. The average particle size of the oil phase means the size of the discontinuous phase in the present application, that is, the diameter of the oil phase in O/W or the oil phase in W/O/W. With such an average particle size of the oil phase, a favorable mouthfeel, appearance, tactile feeling, viscosity, stability, and the like can be achieved. The average particle size of the oil phase is usually larger than 0.5 µm, more preferably not less than 1 µm, still more preferably not less than 2.2 µm, still more preferably not less than 5 µm, still more preferably not less than 10 µm, still more preferably not less than 15 µm. Although there is no upper limit of the average particle size, it is usually not more than 1000 µm, preferably not more than 500 µm, more preferably not more than 250 µm, still more preferably not more than 100 µm, still more preferably not more than 50 µm, still more preferably not more than 40 µm, most preferably not more than 30 µm.

Such an emulsion structure can be confirmed by observation under a polarization microscope. The size of the discontinuous phase means the average size of the longer diameters of discontinuous phases that can be found by observation under a polarization microscope. The number of discontinuous phases observed may be not less than 10, may be not less than 20, may be not less than 40, may be not less than 50, may be not less than 100, or may be not less than 200.

In the present invention, the size of the discontinuous phase of the oil-in-water Pickering emulsion, that is, the particle size distribution, the median size, and the average particle size of the oil phase in the O/W, is measured using a laser diffraction/scattering particle size distribution measuring apparatus.

The content of the oil phase component in the oil-in-water Pickering emulsion according to the present embodiment is not limited as long as the Pickering emulsion can be formed therewith. The content is usually not less than 0.1% by weight, preferably not less than 1% by weight, more preferably not less than 5% by weight, still more preferably not less than 10% by weight, especially preferably not less than 20% by weight, most preferably not less than 30% by weight, and is usually less than 80% by weight, preferably not more than 70% by weight, more preferably not more than 60% by weight, still more preferably not more than 50% by weight, with respect to the total amount of the Pickering emulsion.

### (Aqueous Phase Component)

The aqueous phase component contained in the Pickering emulsion according to the present embodiment is not limited as long as it is a component normally included in emulsions and capable of forming an aqueous phase. The aqueous phase component may contain not only water, but also a lower alcohol, polyol, or the like.

The content of the aqueous phase component in the oil-in-water Pickering emulsion according to the present embodiment is not limited as long as the Pickering emulsion can be formed therewith. The content is usually not less than 20% by weight, preferably not less than 30% by weight, more preferably not less than 40% by weight, especially preferably not less than 50% by weight, and is usually less than 95% by weight, preferably not more than 90% by weight, more preferably not more than 80% by weight, still more preferably not more than 70% by weight, with respect to the total amount of the Pickering emulsion.

### (Other Components)

The Pickering emulsion in the present embodiment may also contain a coloring agent, an antioxidant, a sweetener, a stabilizer, a milk component, a flavoring agent, a coloring agent, a salt, an organic acid, or the like as long as the effect of the present invention is not deteriorated.

Examples of the sweetener include the following.

Sugars: monosaccharides such as glucose, fructose, xylose, sorbose, galactose, and isomerized sugars; disaccharides such as sucrose, maltose, lactose, isomerized lactose, and palatinose; oligosaccharides such as fructo-oligosaccharides, malto-oligosaccharides, isomalto-oligosaccharides, galacto-oligosaccharides, coupling sugar, and palatinose; and the like.

Sugar alcohols: monosaccharide alcohols such as erythritol, sorbitol, xylitol, and mannitol; disaccharide alcohols such as maltitol, isomaltitol, and lactitol; trisaccharide alcohols such as maltotriitol, isomaltotriitol, and panitol; tetra- and higher-saccharide alcohols such as oligosaccharide alcohols; powder reduced maltose syrups; and the like.

High-sweetness sweeteners: aspartame, neotame, sucralose, stevia, and the like.

Examples of the stabilizer include galactomannan, xanthan gum, carrageenan, gum arabic, tamarind gum, gellan gum, glucomannan, and cellulose.

Examples of the milk component include liquid products such as cow milk, processed milk, skimmed milk, fresh cream, whey, butter milk, sweetened condensed milk, and sugar-free condensed milk; and powdered milk products such as whole milk powder, skimmed milk powder, modified milk powder, powdered cream, powdered whey, and butter milk powder. The milk component is especially preferably butter milk or butter milk powder. The butter milk means a liquid component called either butter milk or butter serum which is separated when the milk fat portion is removed as butter by churning or the like from cream produced by centrifugation or the like of cow milk. Examples of the butter milk include condensed butter milk, which is a liquid obtained by condensation of the butter milk, and butter milk powder, which is a powder obtained by spray drying of condensed butter milk. These may be used individually, or two or more of these may be used as a mixture. In some cases, the process of separating cream or butter from cow milk also includes fermentation by an acid-producing bacterium or addition of an acid such as an organic acid. The butter milk used in the present invention is preferably a butter milk obtained without carrying out such fermentation or acid addition.

As the butter milk, a commercially available product such as "Butter Milk Powder", manufactured by Yotsuba Milk Products Co., Ltd., may be used.

As described above, from the viewpoint of avoiding inclusion of allergenic substances in the oil-in-water Pickering emulsion, the milk component is preferably free of casein and β-lactoglobulin, which are highly allergenic. The milk component is more preferably free of milk-derived protein. When casein or β-lactoglobulin is used, its molecular weight is preferably sufficiently reduced by hydrolysis using an enzyme, an acid, or the like in advance such that the casein or β-lactoglobulin exhibits no allergenicity.

The flavoring agent may be an arbitrary flavoring agent. Examples of the flavoring agent include vanilla flavorings such as vanilla essence; and milk flavorings such as milk flavor and butter flavor. The flavoring agent is preferably a milk flavoring. The milk flavoring is not limited as long as it is a flavoring containing a milk aroma component, or a flavoring containing a flavor component characteristic to milk. The milk flavoring may be chemically synthesized, may be extracted or purified from milk, or may be a mixture thereof. The milk flavoring is more preferably prepared using milk as a raw material, still more preferably produced by reacting an enzyme with a milk component, from the viewpoint of reproducing a natural flavor of milk. These may be used individually, or two or more of these may be used as a mixture.

The coloring agent may be an arbitrary coloring agent. Examples of the coloring agent include cacao pigment, β-carotene, annatto pigment, red pepper pigment, turmeric pigment, oil red pigment, paprika pigment, naphthol yellow pigment, and riboflavin butyrate (VB2).

Examples of the salt include chlorides such as common salt, potassium chloride, and magnesium chloride; carbonates such as sodium carbonate, potassium carbonate, and calcium carbonate; bicarbonates such as sodium bicarbonate; phosphates such as disodium phosphate, trisodium phosphate, dipotassium phosphate, and tripotassium phosphate; sodium polyphosphate; citrates such as sodium citrate; and sodium lactate. The salt is especially preferably a salt containing magnesium. Examples of the magnesium salt include those which can be used for application to food, such as milk serum mineral, magnesium chloride, magnesium oxide, magnesium carbonate, magnesium sulfate, bittern (crude magnesium chloride from seawater), dolomite, unrefined salt, magnesium stearate, magnesium hydrogen phosphate, trimagnesium phosphate, magnesium silicate, magnesium hydroxide, magnesium acetate, magnesium citrate, magnesium malate, magnesium benzoate, magnesium gluconate, magnesium L-glutamate, sepiolite, talc, and phytin.

Examples of the organic acid include fumaric acid, succinic acid, citric acid, tartaric acid, diacetyl tartaric acid, malic acid, adipic acid, glutaric acid, and maleic acid.

### (Production of Oil-in-Water Pickering Emulsion)

The oil-in-water Pickering emulsion in the present embodiment may be produced by a per se known method. Examples of the known production method include a method in which the solid particles, nonionic amphiphilic substance, oil phase component, aqueous phase component, and, when necessary, other components, are mixed together, and the resulting mixture is stirred using an arbitrary stirring apparatus.

More specifically, although the method is not limited, the oil-in-water Pickering emulsion may be obtained by the following method:
a production method including: an A1 step of mixing the aqueous phase component with the nonionic amphiphilic substance and the solid particles, and then stirring the resulting mixture; and an A2 step of mixing the mixture obtained in the above step with the oil phase component, and then stirring the resulting mixture; or
a production method including: an A1' step of mixing the oil phase component with the nonionic amphiphilic substance and the solid particles, and then stirring the resulting mixture; and an A2' step of mixing the mixture obtained in the above step with the aqueous phase component, and then stirring the resulting mixture.

The A1 step is a step of preparing an aqueous phase. By preparing the aqueous phase by adding the nonionic amphiphilic substance and the solid particles in combination to the aqueous phase, the amphiphilic substance and the solid particles are allowed to interact with each other, so that the Pickering emulsion can be easily formed. The A1' step is a step of preparing an oil phase. By preparing the oil phase by adding the nonionic amphiphilic substance and the solid particles in combination to the oil phase, the amphiphilic substance and the solid particles are allowed to interact with each other, so that the Pickering emulsion can be easily formed.

The stirring of the mixture in the A1 step or the A1' step may be carried out at normal temperature and pressure, or may be carried out in a warmed state and/or high-pressure state. Although the stirring rate and the stirring time are not limited, the stirring rate is usually 10 rpm to 20,000 rpm, and the stirring time is usually 10 seconds to 5 hours. The stirring rate and/or stirring time may be changed in a stepwise manner.

Examples of stirring apparatus include high-pressure emulsifiers, paddle mixers, homogenizers, ultrasonic homogenizers, colloid mills, kneaders, in-line mixers, static mixers, Onlator, and homomixers. From the viewpoint of enabling sufficient stirring at low energy and low cost, homomixers, paddle mixers, and homogenizers are preferred. A homomixer is more preferably used since it forms a wide convection area to enable uniform stirring of the whole mixture. A combination of different stirring apparatuses may also be used.

The A2 step and the A2' step are steps of preparing a Pickering emulsion. The stirring of the mixture in the A2 step is typically carried out in a warmed state at usually 10°C to 100°C, preferably 20°C to 90°C, more preferably 30°C to 90°C, still more preferably 40°C to 90°C, especially preferably 50°C to 90°C, most preferably 60°C to 90°C, so as to sufficiently melt the oily component. The stirring rate may be usually 10 rpm to 20,000 rpm, and the stirring time is usually 10 seconds to 60 minutes.

The stirring conditions are not limited, and, by changing the stirring rate and/or stirring time in a stepwise manner, a more stable Pickering emulsion can be formed. More specifically, in the first step, oil droplets are dispersed into fine droplets by high-speed stirring, and, in the second step, stirring is carried out at a rate lower than that of the stirring in the first step. This promotes adsorption of the solid particles to the oil-water interface, enabling stabilization of emulsion as a result. The stirring in the second step enables suppression of poor adsorption of the solid particles to the oil-water interface due to the apparatus-derived shear force generated during the high-speed stirring in the first step, and also enables suppression of desorption of the solid particles that have once adsorbed to the interface.

In cases where the stirring conditions are changed in a stepwise manner, the stirring rate in the first step is usually not less than 3000 rpm, more preferably not less than 5000 rpm, still more preferably not less than 7000 rpm, especially preferably not less than 8000 rpm. Although there is no upper limit of the stirring rage, it is usually not more than 25,000 rpm, preferably not more than 20,000 rpm, more preferably not more than 18,000 rpm, still more preferably not more than 16,000 rpm, still more preferably not more than 14,000 rpm, especially preferably not more than 12,000 rpm, most preferably not more than 10,000 rpm. The stirring time in the first step is usually not less than 30 seconds, preferably not less than 1 minute. Although there is no upper limit of the stirring time, it is usually not more than 1 hour, preferably not more than 30 minutes, more preferably not more than 15 minutes, especially preferably not more than 5 minutes.

In cases where the stirring conditions are changed in a stepwise manner, the stirring rate in the second step may be usually not less than 10 rpm, preferably not less than 100 rpm, not less than 500 rpm, not less than 1000 rpm, not less than 2000 rpm, or not less than 2500 rpm. Although there is not upper limit of the rate, it may be usually not more than 10,000 rpm, preferably not more than 8000 rpm, not more than 6000 rpm, or not more than 3000 rpm. Although the stirring time is not limited, it is usually not less than 30 seconds, preferably not less than 1 minute, more preferably not less than 10 minutes, still more preferably not less than 20 minutes, from the viewpoint of promoting adsorption of the solid particles to the oil-water interface.

After the preparation of the oil-in-water Pickering emulsion, sterilization treatment may be carried out at usually not less than 60°C, preferably not less than 65°C, more preferably not less than 70°C, still more preferably not less than 75°C, still more preferably not less than 80°C, especially preferably not less than 95°C, most preferably not less than 100°C, and usually not more than 160°C, preferably not more than 150°C, for usually not less than 0.01 minutes, preferably not less than 0.03 minutes, and usually not more than 60 minutes, preferably not more than about 30 minutes. The method of the sterilization is not limited, and examples thereof include UHT sterilization, retort sterilization, and Joule sterilization. The UHT sterilization may be carried out by a known method such as the direct heating method, for example, the steam injection method, in which water vapor is directly blown to a composition, or the steam infusion method, in which a composition is sprayed into steam to heat the composition; or the indirect heating method, which uses a surface heat exchanger such as a plate-type or tube-type exchanger. For example, a plate-type sterilization apparatus may be used therefor.

The second embodiment of the present description is in accordance with the present invention and described below.

The oil-in-water Pickering emulsion (oil-in-water emulsion composition) according to the second embodiment of the present invention is an oil-in-water Pickering emulsion comprising a solid particle, an oil phase component, an aqueous phase component, and an amphiphilic substance, the solid particle being a rice-derived protein.

In the present description, the oil-in-water Pickering emulsion includes not only the so-called O/W oil-in-water Pickering emulsions, wherein an aqueous phase is present as the continuous phase, but also multiphase emulsions such as W/O/W Pickering emulsions.

### (Oil Phase Component, Aqueous Phase Component, and Solid Particle)

The oil phase component and the aqueous phase component in the second embodiment of the present invention may be the same as the oil phase component and the liquid phase component, respectively, contained in the Pickering emulsion according to the first embodiment of the present description, and the same preferred modes are applicable thereto.

The rice-derived protein in the second embodiment may also be the same as the rice-derived protein in the first embodiment.

### (Amphiphilic Substance)

The amphiphilic substance in the second embodiment of the present invention may be either a nonionic amphiphilic substance or an ionic amphiphilic substance.

The nonionic amphiphilic substance may be the same as the nonionic amphiphilic substance contained in the Pickering emulsion according to the first embodiment of the present description, and the same preferred modes are applicable thereto.

The ionic amphiphilic substance is usually a substance having an ionic structure and an amphiphilic structure in the molecule. The ionic amphiphilic substance is preferably an amphiphilic and surface-active substance, and examples of the substance include amphiphilic polymers, proteins, phospholipids, surfactants, and the like containing a hydrophobic segment and a hydrophilic segment. The ionic amphiphilic substance is preferably a low molecular weight surfactant, and its molecular weight is preferably not more than 5000, more preferably not more than 3000, most preferably not more than 2000. The lower the molecular weight of the low molecular weight surfactant, the larger the number of moles thereof per weight, which is preferred since a larger number of molecules can contribute to the reaction with the solid particles. The lower limit of the molecular weight of the low molecular weight surfactant is not limited. Since a hydrophilic portion and a lipophilic portion are contained in the molecular structure, the molecular weight is usually not less than 200.

In cases where the surfactant contains an alkyl group as a lipophilic group, the alkyl group may be either a linear alkyl group or a branched alkyl group. The alkyl group is preferably a linear alkyl group. Regarding the chain length of the alkyl group, the alkyl group has usually not less than 8, preferably not less than 10, more preferably not less than 12, still more preferably not less than 14, most preferably not less than 16 carbon atoms. Although there is no upper limit of the number of carbon atoms, it is usually not more than 24, preferably not more than 22.

The lipophilic group of the surfactant may also be an alkenyl group or an alkynyl group, which has an unsaturated bond. The presence or absence of branching, chain length, and preferred modes of these groups are the same as those in the case of an alkyl group. In the present embodiment, the lipophilic group is preferably an alkyl group, which has no unsaturated bond.

In the present embodiment, the low molecular weight surfactant does not contain a macromolecule such as protein, polysaccharide, or synthetic polymer.

The ionic amphiphilic substance may be in any form including powder, solid, liquid, or paste.

The ionic amphiphilic substance may be an anionic amphiphilic substance, a cationic amphiphilic substance, an amphoteric amphiphilic substance, or the like.

Examples of the anionic amphiphilic substance include N-acylamino acid salt; alkyl sulfates such as sodium lauryl sulfate; polyoxyethylene alkyl ether sulfates such as sodium polyoxyethylene lauryl ether sulfate; alkyl sulfate ester salts such as triethanolamine lauryl sulfate; sodium stearoyl methyl taurine; triethanolamine dodecylbenzene sulfonate; sodium tetradecene sulfonate; polyoxyethylene lauryl ether phosphoric acid and salts thereof; and N-lauroyl glutamic acid and salts thereof.

Examples of the cationic amphiphilic substance include ammonium-based cationic surfactants and sulfate-based cationic surfactants. Among quaternary ammonium salts, specific examples of the cationic amphiphilic substance include alkyltrimethylammonium salts such as butyltrimethylammonium chloride, hexyltrimethylammonium chloride, octyltrimethylammonium chloride, decyltrimethylammonium chloride, dodecyltrimethylammonium chloride, tetradecyltrimethylammonium chloride, hexadecyltrimethylammonium chloride, stearyltrimethylammonium chloride, butyltrimethylammonium chloride, hexyltrimethylammonium bromide, octyltrimethylammonium bromide, decyltrimethylammonium bromide, dodecyltrimethylammonium bromide, tetradecyltrimethylammonium bromide, hexadecylammonium bromide, and stearyltrimethylammonium bromide. Other examples of the cationic amphiphilic substance include tertiary amidoamines such as stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and diethylaminoethylstearamide.

Examples of the amphoteric amphiphilic substance include phospholipids such as lysolecithin (enzyme-treated lecithin) and lecithin.

In the present embodiment, the amphiphilic substance preferably contains, but does not necessarily need to contain, a functional group capable of reacting or interacting with the surface of the solid particles. By adsorption of the amphiphilic substance to the solid particles through such a functional group, the surface properties of the solid particles can be modified. This enables reinforcement of adsorption of the solid particles to the aqueous phase-oil phase interface, resulting in production of a Pickering emulsion having better emulsion stability than a Pickering emulsion in which the solid particles are used alone.

Examples of the reaction or the interaction between the solid particles and the amphiphilic substance include electrostatic interaction, hydrophobic interaction, intermolecular force interaction, hydrogen bonding, and antigen-antibody reaction. Examples of the functional group which is contained in the amphiphilic substance and which realizes the reaction or the interaction include alkyl groups, cationic groups, anionic groups, amino acid residues, hydroxyl group, carboxyl group, peptides, proteins, and antigens. From the viewpoint of inhibition of electrostatic interaction by salts, hydrophobic interaction and/or hydrogen bonding is/are preferred. In cases where the solid particles are hydrophobic proteins, hydrophobic interaction is preferred.

The method of analysis of the amphiphilic substance contained in the oil-in-water Pickering emulsion is not limited. For example, the analysis may be carried out by the same method as the method described for the first embodiment.

The content of the amphiphilic substance in the oil-in-water Pickering emulsion according to the present embodiment is not limited as long as in a range where the amphiphilic substance can be contained in the Pickering emulsion. The content is usually not less than 0.00001% by weight, preferably not less than 0.00005% by weight, more preferably not less than 0.0001% by weight, most preferably not less than 0.001% by weight, and is usually not more than 5% by weight, preferably less than 1% by weight, more preferably not more than 0.5% by weight, still more preferably not more than 0.1% by weight, especially preferably not more than 0.05% by weight, most preferably not more than 0.01% by weight, with respect to the total amount of the Pickering emulsion.

The weight of the amphiphilic substance with respect to the weight of the solid particles (nonionic amphiphilic substance/solid particles) is usually not less than 0.00001, preferably not less than 0.00005, more preferably not less than 0.0001, still more preferably not less than 0.0005, especially preferably not less than 0.001, most preferably not less than 0.0025, and is usually not more than 5, preferably less than 1, more preferably not more than 0.5, still more preferably not more than 0.1, still more preferably not more than 0.05, especially preferably not more than 0.01**,** most preferably not more than 0.005.

The concentration of the amphiphilic substance in the oil-in-water Pickering emulsion in the present embodiment is more preferably not more than the critical micelle concentration. In cases where the concentration of the amphiphilic substance is not more than the critical micelle concentration, the amphiphilic substance is capable of binding or adsorbing to the solid particles to form a single layer without forming micelles. Thus, the surface properties of the solid particles can be efficiently modified, and as a result, the amount of the amphiphilic substance added can be reduced.

In the present embodiment, a nonionic amphiphilic substance is preferably used as the amphiphilic substance. This is because nonionic amphiphilic substances are less likely to be affected by pH and salts than ionic amphiphilic substances, and hence applicability of nonionic amphiphilic substances is less likely to be limited due to the solid particles, the oil phase component, the liquid phase component, or other components.

### (Other Components)

The oil-in-water Pickering emulsion in the present embodiment may also contain a coloring agent, an antioxidant, a sweetener, a stabilizer, a milk component, a flavoring agent, a coloring agent, a salt, an organic acid, or the like as long as the effect of the present invention is not deteriorated. The same other components as those contained in the Pickering emulsion according to the first embodiment of the present description may be used, and the same preferred modes are applicable thereto.

### (Production of Oil-in-Water Pickering Emulsion)

The oil-in-water Pickering emulsion in the present embodiment may be produced by the same method as the method for the Pickering emulsion according to the first embodiment of the present description, and the same preferred modes are applicable thereto.

### (Structure of Oil-in-Water Pickering Emulsion)

The oil-in-water Pickering emulsions in the first and second embodiments of the present description have an emulsion structure in which the solid particles are present on the interface between the oil phase component and the aqueous phase component. By having such a structure, the oil-in-water Pickering emulsion can have a controlled particle size both before and after heating, and can have heat resistance and cooling resistance.

The structure in which the solid particles are present on the interface between the oil phase component and the aqueous phase component means a structure in which the solid particles are adsorbed on the interface between the oil phase component and the aqueous phase component. By this, the oil phase can be emulsified in the aqueous phase to allow formation of the so-called Pickering emulsion. More specifically, this structure is a structure in which at least part of the solid particles is adsorbed on the surface of the oil phase emulsified in the aqueous phase.

The presence of the solid particles on the interface between the oil phase component and the aqueous phase component can be confirmed by observation of a cross-section of the oil-in-water Pickering emulsion using a cryo-scanning electron microscope (cryo-SEM) or the like. The method of observing the cross-section is not limited as long as it is a method normally used. For example, the oil-in-water Pickering emulsion may be quickly frozen by the metal contact method or the like, and then the frozen Pickering emulsion may be cut using a cryomicrotome with a diamond knife for light microscopy, to prepare a sample. A cross-section of the sample may then be observed by cryo-SEM.

Since the oil-in-water Pickering emulsions in the first and second embodiments have the above structure, they have emulsion stability which allows storage for a longer period. Further, the presence of the above structure enables better suppression of oxidative deterioration of oils and fats, and of the progress of hydrolysis, thereby suppressing generation of the characteristic odor during the emulsification, or during storage of the emulsion composition after the emulsification.

### (pH of Oil-in-Water Pickering Emulsion)

The pH of the oil-in-water Pickering emulsion is not limited, and a preferred pH may be selected depending on the intended use. For example, in uses for food, the pH is not limited as long as the food is edible or drinkable. Regarding the lower limit, the pH is usually more than 1, preferably not less than 2, more preferably not less than **3,** still more preferably not less than 4, still more preferably not less than 5, especially preferably not less than 5.5, most preferably not less than 6. Regarding the upper limit, the pH is usually not more than 13, preferably not more than 12, more preferably not more than 10, still more preferably not more than 9, especially preferably not more than 8, most preferably not more than 7.5. When a protein is used as the solid particles, the pH of the oil-in-water emulsion may be near the isoelectric point of the protein which is a major component constituting the solid particles. However, from the viewpoint of avoiding generation of coarse aggregates or precipitates, the pH is usually different from the isoelectric point by at least 0.5, more preferably by at least 1.

### (Use of Oil-in-Water Pickering Emulsion)

The oil-in-water Pickering emulsions of the first and/or second embodiment(s) of the present description may be used for pharmaceuticals; cosmetics; food; feeds; diagnostic agents; carriers for drug delivery systems (DDS); washing agents; coating agents; surface treatment agents; toiletry products; personal care products; and the like. They may be used for, for example, oral ingestion or transdermal absorption. They may be used also in the forms of intermediate products.

In cases where the oil-in-water Pickering emulsions are used for oral ingestion, their products, uses, properties, and the like are not limited as long as they are orally ingested. Specific examples of uses of the oil-in-water Pickering emulsions include food and beverages such as beverages, liquid food, cream food, and milk substitutes; retort processed dietary supplements; functional food such as liquid diet; oral vaccines; wheat flour products such as bread and noodle; oil and fat processed products such as fat spread and flour paste; sauces and soups such as curry, coffee creamer, mayonnaise, dressing, mousse, pasta sauce, stew, demi-glace sauce, white sauce, and tomato sauce; retort processed food and composite seasonings such as seasoning mixtures for Chinese dishes or rice bowls; confectionery and dessert such as yogurts, cheese, ice creams, creams, caramel, candy, chewing gum, chocolate, cookie, biscuit, cake, pie, snack, cracker, Japanese confectionery, rice confectionery, bean confectionery, jelly, and pudding; processed livestock products such as hamburg steak, meatball, and canned seasoned meat; frozen food; refrigerated food; cooked or semi-cooked food such as packed or in-store prepared daily dishes; instant food such as instant noodle, cup noodle, and instant soups and stews; fortified diet; food and beverages such as liquid diet, high-calorie diet, and infant nutrition products; and tube feeding formulae.

Beverages and liquid food are especially preferred. Examples of the beverages include milk beverages, soup beverages, coffee beverages, cocoa beverages, tea beverages (such as black tea, green tea, and Chinese tea), bean or cereal beverages, and acidic beverages. Among these, milk beverages, coffee beverages, and tea beverages are preferred. The milk substitute means an emulsion composition that may substitute an animal-derived milk such as cow milk from the viewpoint of taste, flavor, and physical properties. The oil-in-water Pickering emulsions may also be used in the forms of intermediate products for food such as yogurt and ice cream. Examples of the physical properties include the particle size distribution of the oil droplets in the composition, the viscosity, the pH, the stability of the emulsion, and the appearance. Oil-in-water Pickering emulsions for food in the present embodiment may be favorably used for canned beverages, plastic bottled beverages, carton beverages, glass bottled beverages, and the like.

### EXAMPLES

The present invention is described below in more detail by way of Examples. Needless to say, however, the scope of the present invention is not limited to the modes described in the following Examples.

### <Measurement of Contact Angle of Water on Solid Particle>

Solid particles were prepared into a tablet, and then water was dropped thereon by its own weight. The contact angle was then measured over time. In order to minimize the effect of liquid absorption into the rough or porous surface of the tablet, linear approximation was carried out using measurement values with which the change in the contact angle became almost linear with respect to the time after the attachment of the droplet (t), to calculate the contact angle at the time of the droplet attachment (t = 0), which was provided as the contact angle of water on the solid particle.

The preparation of the tablet of solid particles (tablet molding) was carried out using a tablet press (for 20-mm diameter). The solid particles were placed in an internal cylinder. After pressurization to 5 tons, the pressure was reduced using a vacuum pump, and then pressure was applied to 8 tons and then 10 tons in a stepwise manner to perform molding. Using 0.23 g of solid particles, a sample for measurement of the contact angle was prepared.

For the measurement of the contact angle, FTÅ (First Ten Ångstroms (USA)) was used. When the contact angle of water was measured, about 12 to 13 µL of water was dropped by its own weight on the tablet molded as described above, and the contact angle after the attachment of the droplet was measured over time.

The measurement of the contact angle was carried out at 23°C at a humidity of 50%.

### <Preparation of Oil-in-Water Emulsions>

Oil-in-water emulsions were prepared using rice-derived protein (ORYZA POTEIN^{™}-P70, manufactured by Oryza Oil & Fat Chemical Co., Ltd; protein obtained from seeds of a gramineous plant *(Oryza sativa* Linne)) as solid particles; a sucrose fatty acid ester (RYOTO Sugar Ester S-570, RYOTO Sugar Ester S-1170, or RYOTO Sugar Ester S-1670, manufactured by Mitsubishi-Chemical Foods Corporation) as a nonionic amphiphilic substance; hydrogenated coconut oil as an oil phase component; and demineralized water as an aqueous phase component; such that the total amount was 100 parts by weight. Table 1 shows the amount of each component added (part by weight) in each of Examples and Comparative Examples.

ORYZA POTEIN^{™}-P70, RYOTO Sugar Ester S-570, RYOTO Sugar Ester S-1170, and RYOTO Sugar Ester S-1670 may be hereinafter simply referred to as P70, S-570, S-1170, and S-1670, respectively.

The physical properties of the raw materials are as follows.
- P70: amino acid score = 56, contact angle of water = 69°
- S-570: monoester content = 29% by weight, HLB = 5
- S-1170: monoester content = 58% by weight, HLB = 11
- S-1670: monoester content = 78% by weight, HLB = 16
- Hydrogenated coconut oil: slip melting point = 32°C

### (Example 1)

First, rice-derived protein, and an aqueous solution preliminarily prepared by addition of a sucrose fatty acid ester (S-1670), were placed in a container, and then mixed together to obtain an aqueous phase. The aqueous solution of the sucrose fatty acid ester was prepared by dissolution by warming.

Subsequently, the aqueous phase, and liquid hydrogenated coconut oil heated to 60°C were placed in a container, and then the resulting mixture was further warmed to 60°C. The resulting mixture was stirred using a homogenizer (IKA T25 digital ULTRA TURRAX, shaft generator: S25N-10G) at 10,000 rpm for 2 minutes, to obtain oil-in-water Pickering emulsion A.

### (Example 2)

The same operation as in Example 1 was carried out except that S-1170 was used instead of S-1670, to obtain oil-in-water Pickering emulsion B.

### (Example 3)

The same operation as in Example 1 was carried out except that S-570 was used instead of S-1670, to obtain oil-in-water Pickering emulsion C.

### (Comparative Example 1)

The same operation as in Example 1 was carried out except that S-1670 was not used, to obtain oil-in-water Pickering emulsion D.

### (Comparative Example 2)

The same operation as in Example 1 was carried out except that P70 was not used, to obtain oil-in-water Pickering emulsion E.

### (Comparative Example 3)

The same operation as in Example 2 was carried out except that P70 was not used, to obtain oil-in-water Pickering emulsion F.

### (Comparative Example 4)

The same operation as in Example 3 was carried out except that P70 was not used, to obtain oil-in-water Pickering emulsion G.

**Table 1**

| | | Example 1 | Example 2 | Example 3 | Comparative Example 1 | Comparative Example 2 | Comparative Exmple 3 | Comparative Example 4 |
|---|---|---|---|---|---|---|---|---|
| Aqueous phase | Demineralized water | 68.5965 | 68.5965 | 68.5965 | 68.5965 | 68.5965 | 68.5965 | 68.5965 |
| | P70 (parts by weight) | 1.4 | 1.4 | 1.4 | 1.4 | 0 | 0 | 0 |
| | S-1670 (parts by weight) | 3.5×10³ | 0 | 0 | 0 | 3.5×10³ | 0 | 0 |
| | S-1170 (parts by weight) | 0 | 3.5×10⁻³ | 0 | 0 | 0 | 3.5×10⁻³ | 0 |
| | S-570 (parts by weight) | 0 | 0 | 3.5×10⁻³ | 0 | 0 | 0 | 3.5×10⁻³ |
| Oil phase | Hydrogenated coconut oil (parts by weight) | 30 | 30 | 30 | 30 | 30 | 30 | 30 |
| Total amount (parts by weight) | | 100 | 100 | 100 | 100 | 100 | 100 | 100 |
| Amphiphilic substances/Solid particles | | 0.0025 | 0.0025 | 0.0025 | - | - | - | - |

### <Evaluation of Cooling Stabilities of Oil-in-Water Emulsions 1>

Each of the oil-in-water emulsions according to Examples 1 to 3 and Comparative Examples 1 to 4 was emulsified at 60°C, and then placed in a container, followed by immersing the container in a water bath for not less than 30 minutes to thereby cool the container to 25°C. Thereafter, the container was inverted, and vibration was applied to the container. The fluidity and the presence or absence of aggregation of the oil-in-water emulsion before and after the cooling were visually observed. The evaluation results are shown in Table 2. The evaluation criteria are as follows.
Good: No aggregates in the emulsion; good fluidity.
Poor: Generation of aggregates in the emulsion; poor fluidity.

**Table 2**

| | Example 1 | Example 2 | Example 3 | Comparative Example 1 | Comparative Example 2 | Comparative Example 3 | Comparative Example 4 |
|---|---|---|---|---|---|---|---|
| Before cooling (60°C) | Good | Good | Good | Good | Good | Good | Good |
| After cooling (25°C) | Good | Good | Good | Poor | Poor | Poor | Poor |

As is evident from the results shown in Table 2, oil-in-water Pickering emulsions A to C, which were prepared by adding a sucrose fatty acid ester and rice-derived protein in combination, showed no aggregation, and were capable of flowing, even after the cooling. Thus, a stable emulsion state could be maintained.

### <Evaluation of Cooling Stabilities of Oil-in-Water Emulsions 2>

Oil-in-water Pickering emulsion A (Example 1) cooled to room temperature was observed using a polarization microscope ECLIPSE LV100N POL, manufactured by Nikon Corporation, and image integration software NIS-Elements Ver 3.2, manufactured by Nikon Corporation.

In an image for an area of 464 × 623 µm, 40 oil droplets were arbitrarily selected, and the oil droplet diameters were measured. The average oil droplet diameter was 27.98 µm, and the standard deviation was 6.98 µm.

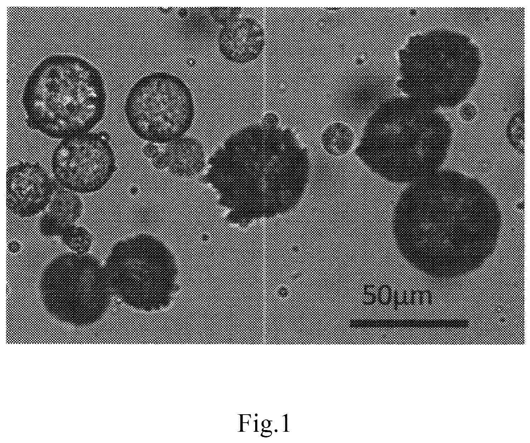

Oil-in-water Pickering emulsion A cooled to room temperature was diluted 10-fold with demineralized water, and observed using a polarization microscope. As a result, the presence of solid particles on the emulsion surface layer could be confirmed (Fig. 1). According to this result, it was found that, in cases where an aqueous phase is prepared using rice-derived protein and a sucrose fatty acid ester in combination, and then emulsification is carried out, the resulting emulsion has a structure in which the rice-derived protein is present on the aqueous phase-oil phase interface even after cooling. It was thus found that Pickering emulsion A can be stably present as an oil-in-water Pickering emulsion maintaining emulsion stability even after cooling, indicating that it has high cooling stability.

### <Evaluation of Aqueous Phase-Oil Phase Interfacial Breakage>

Observation was carried out in the extinction position using the polarization microscope that was used for the evaluation of the cooling stability. As a result, the internal phase portion of the emulsion appeared as a bright spot, so that occurrence of crystallization of the hydrogenated coconut oil in the emulsion internal phase was confirmed. Thus, the absence of needle crystals protruding from the oil phase toward the liquid phase was indicated.

### <Evaluation of High-Temperature Stabilities of Oil-in-Water Emulsions>

Each of the oil-in-water Pickering emulsions A and C according to Example 1 and Example 3 was placed in a container, and warmed under the conditions shown in Table 3. Thereafter, the presence or absence of oil phase separation was visually observed from the side of the container. The evaluation results are shown in Table 3. The evaluation criteria are as follows.
Good: No oil phase separation.
Poor: Occurrence of oil phase separation.

**Table 3**

| | | Example 1 | Example 3 |
|---|---|---|---|
| Heating condition | 65°C, 30 min | Good | Good |
| | 75°C, 15 sec | Good | Good |

### <Preparation of Oil-in-Water Emulsions>

Oil-in-water emulsions were prepared using rice-derived protein (ORYZA POTEIN^{™}-P70, manufactured by Oryza Oil & Fat Chemical Co., Ltd; protein obtained from seeds of a gramineous plant *(Oryza sativa* Linne)) as solid particles; a sucrose fatty acid ester (RYOTO Sugar Ester S-1670, manufactured by Mitsubishi-Chemical Foods Corporation) as a nonionic amphiphilic substance; hydrogenated coconut oil as an oil phase component; and demineralized water as an aqueous phase component; such that the total amount was 100 parts by weight. Table 4 shows the amount of each component added (part by weight) in each of Examples and Comparative Examples.

ORYZA POTEIN^{™}-P70 and RYOTO Sugar Ester S-1670 may be hereinafter simply referred to as P70 and S-1670, respectively.

The physical properties of the raw materials are as follows.
- P70: amino acid score = 56, contact angle of water = 69°
- S-1670: monoester content = 78% by weight, HLB = 16
- Hydrogenated coconut oil: slip melting point = 32°C

### (Example 4)

First, rice-derived protein, and an aqueous solution containing a sucrose fatty acid ester (S-1670), were placed and mixed in a container such that the weight ratio, in terms of the amphiphilic substance/the solid particles, was as described in Table 4. The resulting mixture was left to stand to allow precipitation of coarse aggregates of the rice-derived protein, and then the resulting supernatant was collected. From the dry weight of the supernatant collected, the solid content concentration was calculated, and, based on the weight ratio in terms of the amphiphilic substance/the solid particles, the protein concentration was calculated. The supernatant collected was diluted with water to a desired concentration, to obtain an aqueous phase. The aqueous solution of the sucrose fatty acid ester was prepared by dissolution in water with warming.

Subsequently, the aqueous phase, and liquid hydrogenated coconut oil heated to 60°C, were placed in a container, and the resulting mixture was further warmed to 60°C. The mixture was then stirred at 60°C using a homomixer (T.K. Robomix, manufactured by PRIMIX Corporation) at 8000 rpm to 10,000 rpm for 1 minute, and then at 3000 rpm for 20 minutes, to obtain oil-in-water Pickering emulsion H.

### (Example 4')

The same operation as in Example 4 was carried out except that the aqueous phase prepared was subjected to heat treatment at 80°C for 30 minutes, and that different stirring conditions were used, to obtain oil-in-water Pickering emulsion H'. The stirring was carried out at 8000 rpm to 10,000 rpm for 10 minutes, and then at 3000 rpm for 20 minutes.

### (Example 5)

The same operation as in Example 4 was carried out except that the amounts of the rice-derived protein and the sucrose fatty acid ester (S-1670) added were as shown in Table 4, to obtain oil-in-water Pickering emulsion I.

### (Example 6)

The same operation as in Example 4 was carried out except that the amounts of the rice-derived protein and the sucrose fatty acid ester (S-1670) added were as shown in Table 4, to obtain oil-in-water Pickering emulsion J.

### (Example 7)

The same operation as in Example 6 was carried out except that the amount of the sucrose fatty acid ester (S-1670) added was as shown in Table 4, to obtain oil-in-water Pickering emulsion K.

### (Example 8)

The same operation as in Example 6 was carried out except that the amount of the sucrose fatty acid ester (S-1670) added was as shown in Table 4, to obtain oil-in-water Pickering emulsion L.

### (Example 9)

The same operation as in Example 4 was carried out except that the amounts of the rice-derived protein, the preliminary sucrose fatty acid ester (S-1670), and the hydrogenated coconut oil added were as shown in Table 4, to obtain oil-in-water Pickering emulsion M.

**Table 4**

| | Example 4 (Example 4') | Example 5 | Example 6 | Example 7 | Example 8 | Example 9 |
|---|---|---|---|---|---|---|
| Demineralized water (parts by weight) | Remainder | Remainder | Remainder | Remainder | Remainder | Remainder |
| P70 (parts by weight) | 0.40 | 0.050 | 0.099 | 0.099 | 0.099 | 0.031 |
| S-1670 (parts by weight) | 0.99×10⁻³ | 1.2×10⁻⁴ | 2.5×10⁻⁴ | 2.5×10⁻³ | 5.0×10⁻³ | 7.8×10⁻⁵ |
| Amphiphilic substances/solid particles (parts by weight) | 0.0025 | 0.0025 | 0.0025 | 0.025 | 0.050 | 0.0025 |
| Hydrogenated coconut oil (parts by weight) | 1 | 1 | 1 | 1 | 1 | 10 |
| Total amount (parts by weight) | 100 | 100 | 100 | 100 | 100 | 100 |
| Oil phase/aqueous phase (parts by weight) | 1/99 | 1/99 | 1/99 | 1/99 | 1/99 | 10/90 |

### <Evaluation of Cooling Stabilities of Oil-in-Water Emulsions>

Each of the Pickering emulsions according to Examples 4 and 4' was placed in a container, and heated under the conditions shown in Table 5. Thereafter, the presence or absence of oil phase separation was visually observed from the side and top of the container. The evaluation results are shown in the "appearance immediately after heating" column of Table 5. The evaluation criteria are as follows.
Good: No oil phase separation was found by observation from the side and top.
Normal: No oil phase separation was found by observation from the side, but a small number of oil droplets were found by observation from the top.
Poor: Oil phase separation was found by observation from the side.

Subsequently, the container heated under the conditions shown in Table 5 was immersed in a water bath for not less than 30 minutes, to cool the container to 25°C. Thereafter, the container was inverted, and vibration was applied to the container. The fluidity and the presence or absence of aggregation of the Pickering emulsion after the cooling were visually observed. The evaluation results are shown in the "appearance after cooling" column of Table 5. The evaluation criteria are as follows.
Good: No aggregates in the emulsion; good fluidity. Normal: Slight generation of aggregates in the emulsion, but good fluidity.
Poor: Generation of aggregates in the emulsion; poor fluidity.

**Table 5**

| | Heating temperature | Heating time | appearance immediately after heating | appearance after cooling |
|---|---|---|---|---|
| Example 4 (H) | 65°C | 30 min | Good | Good |
| Example 4'(H') | 60°C | 0 min | Good | Good |
| | 65°C | 30 min | Good | Normal |
| | 75°C | 15 sec | Good | Normal |

As is evident from the results, Pickering emulsion H, which was prepared by adding a sucrose fatty acid ester and rice-derived protein in combination, showed no aggregation, and was capable of flowing, even after the cooling. Thus, a stable emulsion state could be maintained.

Pickering emulsion H', which was prepared using a preliminarily heat-treated aqueous phase, showed no oil phase separation after the heating, and had good heat resistance. Moreover, it showed no aggregation, and was capable of flowing, even after the cooling. Thus, a stable emulsion state could be maintained.

Also, by the use of the rice-derived protein denatured by the heat treatment, preparation of a Pickering emulsion having good heat resistance and cooling resistance was possible.

To determine the oil droplet size of the Pickering emulsion H' after the cooling, particle size distribution measurement was carried out by the flow measurement method using a laser diffraction/scattering particle size distribution measuring apparatus LA-920, manufactured by Horiba, Ltd. The results are shown in Table 6. Using the value 1.20 as the relative refractive index, the analysis was carried out in terms of the volume. The value of the relative refractive index was calculated using a refractive index of 1.60 for the oil/fat, and a refractive index of 1.33 for water.

### <Evaluation of Taste>

The Pickering emulsion H according to Example 4 was cooled to room temperature, and then stored under refrigeration. The Pickering emulsion was then subjected to comparison with the following standard beverage at room temperature by drinking by each of four panelists who were researchers engaged in the research and development. By this, comparative evaluation of the intensity of oily feeling was carried out, and then the four researchers had a discussion to obtain a consensus, which is shown in Table 6. The Pickering emulsion H according to Example 4 was subjected to heat sterilization treatment at 65°C for 30 minutes before the cooling to room temperature.

### (Standard Beverage)

A composition containing 0.0443% by mass sucrose fatty acid ester (RYOTO Sugar Ester S-770; HLB = 7; manufactured by Mitsubishi-Chemical Foods Corporation), 0.00443% by mass sucrose fatty acid ester (RYOTO Sugar Ester S-370; HLB = 3; manufactured by Mitsubishi-Chemical Foods Corporation), 0.00443% by mass monoglycerin succinic acid fatty acid ester, and, as an oil phase component, 1% by mass hydrogenated coconut oil, was used as the standard beverage.

The pH was measured with LAQUA PH/ION METER F-72, manufactured by Horiba, Ltd. To determine the oil droplet size, particle size distribution measurement was carried out by the batch measurement method using a laser diffraction/scattering particle size distribution measuring apparatus LA-950, manufactured by Horiba, Ltd. Assuming the refractive index of the substance to be measured (oil or fat) as 1.60, the analysis was carried out.

**Table 6**

| | Example 4' (H') | Example 4 (H) | Standard beverage |
|---|---|---|---|
| oil phase/aqueous phase (parts by weight) | 1/99 | 1/99 | 1/99 |
| pH | - (not determined) | 6.7 | 7.1 |
| Oil droplet size Average particle size in terms of the volume (µm) | 30 | 16 | 0.36 |
| Oil droplet size Median size in terms of the volume (µm) | 26 | 14 | 0.34 |
| Oil droplet size Peak size in terms of the volume (µm) | 1.5, 29 | 0.25, 1.5, 26 | 0.38 |
| Taste | - (not determined) | More intense oily feeling than the standard beverage | - |

The Pickering emulsion H according to Example 4 was cooled to room temperature, and then stored under refrigeration. After storage under refrigeration at 4°C for 8.5 months, the particle size distribution and the pH were measured. The standard beverage was similarly stored under refrigeration, and then evaluated. The results are shown in Table 7.

In the standard beverage, which was prepared according to conventional surfactant-based emulsification, the pH was substantially decreased and the oil droplet size was increased after the storage for 8.5 months. In contrast, in the Pickering emulsion according to the present embodiment, changes in the pH and the oil droplet size were hardly observed after the storage for 8.5 months, indicating that the Pickering emulsion has excellent long-term storage stability. Moreover, in contrast to the standard beverage, which had a fatty acid odor after the storage for 8.5 months, the Pickering emulsion H according to Example 4 had no fatty acid odor after the storage for 8.5 months.

**Table 7**

| | Example 4 (H) | Standard beverage |
|---|---|---|
| Oil phase/aqueous phase (parts by weight) | 1/99 | 1/99 |
| pH after storage for 8.5 months | 6.7 | 4.3 |
| Oil droplet size Average particle size in terms of the volume (µm) | 15 | 35 |
| Oil droplet size Median size in terms of the volume (µm) | 13 | 30 |
| Oil droplet size Peak size in terms of the volume (µm) | 0.33, 1.9, 22 | 29 |

These results indicate that the Pickering emulsion according to the present embodiment can provide a novel beverage which has a more intense oily feeling than that of conventional surfactant-based emulsions, and which has a unique flavor and richness. Further, the Pickering emulsion according to the present embodiment has better long-term storage stability than that of conventional surfactant-based emulsions.

### <Microscopic Observation> (Evaluation of Emulsion Systems)

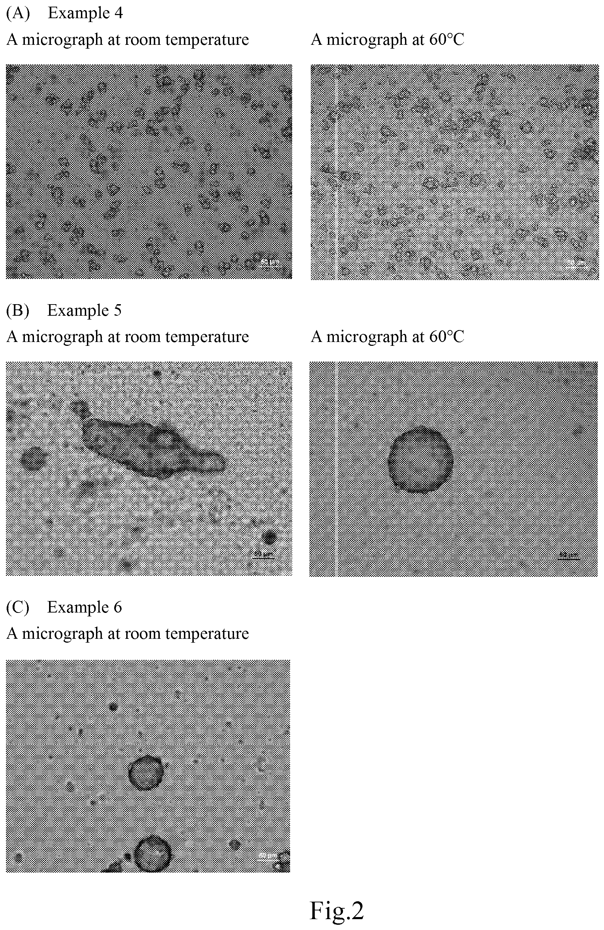

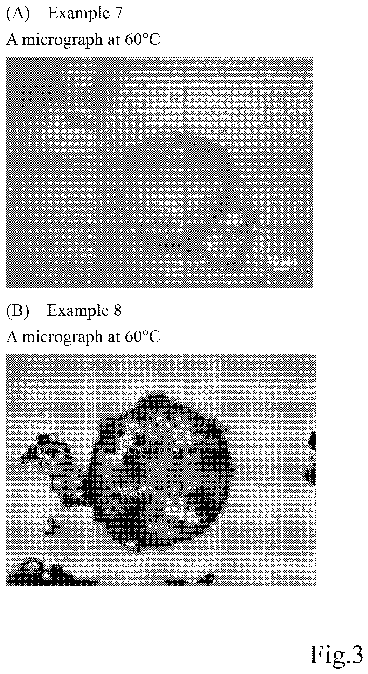

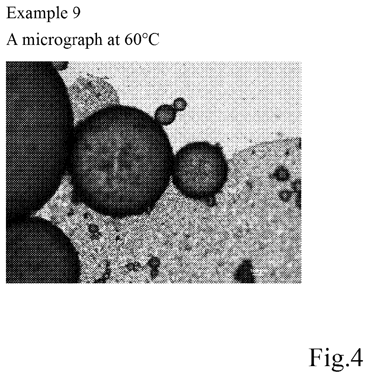

The Pickering emulsions H to M according to Examples 4 to 9 were observed by microscopy using a polarization microscope ECLIPSE LV100N POL, manufactured by Nikon Corporation, and image integration software NIS-Elements Ver 3.2, manufactured by Nikon Corporation. As result, emulsion formation was confirmed for all of these (Figs. 2 to 4). Further, the presence of solid particles (rice-derived protein) on the oil-water interface was confirmed.

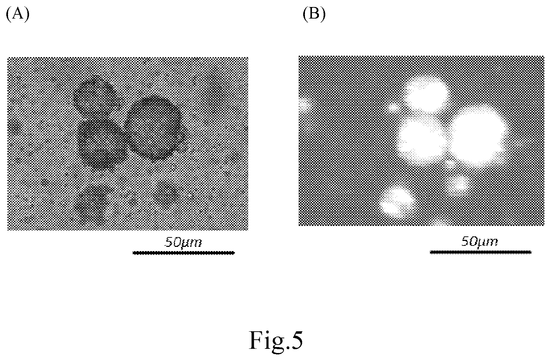

The results of observation of the Pickering emulsion H according to Example 4 using the polarization microscope in the open-Nicol and cross-Nicol configurations are shown in Fig. 5. In the micrograph obtained by the observation in the cross-Nicol configuration, the internal phase portion of the emulsion appeared as a bright spot. It was thus found that crystallization of the hydrogenated coconut oil occurred in the emulsion internal phase, and that there were no protruding needle crystals (Fig. 5).

For micrographs obtained at sizes suitable for observation of the Pickering emulsions (Figs. 2 to 4), the longer diameters of the emulsions having the largest sizes are shown below in Table 8. For the Pickering emulsion of Example 4 (H), the longer diameters were measured at 15 points in a microscopic image (an area of 464 × 623 µm) at 60°C. As a result, the average and the standard deviation were 24.79 ± 6.37 µm. Further, the Pickering emulsion of Example 4 (H) was cooled to 25°C, and the longer diameters were measured at 15 points in its microscopic image (an area of 464 × 623 µm). As a result, the average and the standard deviation were 28.53 ± 5.71 µm.

**Table 8**

| | Example 4 | Example 5 | Example 6 | Example 7 | Example 8 | Example 9 |
|---|---|---|---|---|---|---|
| Demineralized water (parts by weight) | Remainder | Remainder | Remainder | Remainder | Remainder | Remainder |
| P70 (parts by weight) | 0.40 | 0.050 | 0.099 | 0.099 | 0.099 | 0.031 |
| S-1670 (parts by weight) | 0.99×10⁻³ | 1.2×10⁻⁴ | 2.5×10⁻⁴ | 2.5×10⁻³ | 5.0×10⁻³ | 7.8×10⁻⁵ |
| Amphiphilic substances/ solid particles (parts by weight) | 0.0025 | 0.0025 | 0.0025 | 0.025 | 0.050 | 0.0025 |
| Oil phase/aqueous phase (parts by weight) | 1/99 | 1/99 | 1/99 | 1/99 | 1/99 | 10/90 |
| Largest emulsion size observed at room temperature (µm) | 39 | 323 | 80 | - | - | - |
| Largest emulsion size observed at 60°C (µm) | 39 | 146 | - | 123 | 611 | 936 |

From these results, it was found that the higher the content of the solid particles, the smaller the particle size of the emulsion can be. It was also found that the lower the content of the amphiphilic substance, the smaller the particle size of the emulsion can be.

### <Preparation of Oil-in-Water Emulsions>

Oil-in-water emulsions were prepared using a product prepared by subjecting rice-derived protein (ORYZA POTEIN^{™}-P70, manufactured by Oryza Oil & Fat Chemical Co., Ltd; protein obtained from seeds of a gramineous plant *(Oryza sativa* Linne)) to crushing treatment for 4 hours using a paint shaker (PAINT SHAKER, manufactured by TOYOSEIKI) and 1-mm-diameter zirconia beads (hereinafter the product is referred to as crushed protein), as solid particles; a sucrose fatty acid ester (RYOTO Sugar Ester S-1670, manufactured by Mitsubishi-Chemical Foods Corporation) as a nonionic amphiphilic substance; hydrogenated coconut oil as an oil phase component; and demineralized water as an aqueous phase component; such that the total amount was 100 parts by weight. Table 10 shows the amount of each component added (parts by weight) in each of Examples and Comparative Examples.

ORYZA POTEIN^{™}-P70, RYOTO Sugar Ester S-570, RYOTO Sugar Ester S-970, RYOTO Sugar Ester S-1670, RYOTO Polyglycerol Ester SWA-10D, and RYOTO Polyglycerol Ester S-28D may be hereinafter simply referred to as P70, S-570, S-970, S-1670, SWA-10D, and S-28D, respectively.

The physical properties of the raw materials are as follows.
- P70: amino acid score = 56, contact angle of water = 69°
- S-570: monoester content = 29% by weight, HLB = 5
- S-970: monoester content = 55% by weight, HLB = 9
- S-1670: monoester content = 78% by weight, HLB = 16
- SWA-10D: HLB = 14
- S-28D: HLB = 9
- Hydrogenated coconut oil: slip melting point = 32°C

### (Experimental Example: Control of Particle Size of Rice-Derived Protein)

First, a sucrose fatty acid ester (S-1670) was dissolved in demineralized water with warming. To the resulting solution, rice-derived protein was added, and the resulting mixture was mixed to obtain a rice-derived protein dispersion. The rice-derived protein dispersion was prepared such that the ratio of the rice-derived protein was 10% by weight, and such that the ratio of the sucrose fatty acid ester added with respect to 1 part by weight of the rice-derived protein was 0.0025 parts by weight.

To 15 parts by weight of the thus prepared rice-derived protein dispersion, 20 parts by weight of 1-mm-diameter zirconia beads were added, and crushing treatment was carried out using a paint shaker (PAINT SHAKER, manufactured by TOYOSEIKI) for various lengths of treatment time, to obtain crushed protein dispersions. The particle size distribution of the protein particles (solid particles) in each crushed protein dispersion was measured by the batch measurement method using a laser diffraction/scattering particle size distribution measuring apparatus LA-950, manufactured by Horiba, Ltd. Assuming the refractive index of the substance to be measured as 1.60, and the refractive index of water as 1.33, the analysis was carried out in terms of the volume. The results are shown in Table 9.

**Table 9**

| Treatment time using a paint shaker (hours) | Average particle size of protein particles (µm) | Median size of protein particles (µm) |
|---|---|---|
| 0 | 55 | 53 |
| 0.5 | 45 | 35 |
| 1 | 48 | 22 |
| 2 | 31 | 17 |
| 4 | 12 | 11 |
| 6 | 5.8 | 5.4 |

It was found that the particle size distribution of the protein particles (solid particles) in the dispersion medium can be controlled to a desired average particle size by the crushing treatment.

### (Example 10)

First, a sucrose fatty acid ester (S-1670) was dissolved in demineralized water with warming. To the resulting solution, rice-derived protein was added, and the resulting mixture was mixed to obtain a rice-derived protein dispersion. The rice-derived protein dispersion was prepared such that the ratio of the rice-derived protein was 10% by weight, and such that the ratio of the sucrose fatty acid ester added with respect to 1 part by weight of the rice-derived protein was 0.0025 parts by weight.

To 15 parts by weight of the thus provided rice-derived protein dispersion, 20 parts by weight of 1-mm-diameter zirconia beads were added, and crushing treatment was carried out using a paint shaker (PAINT SHAKER, manufactured by TOYOSEIKI) for 4 hours, to obtain a crushed protein dispersion. The crushed protein dispersion and demineralized water were placed in a container, and then mixed together to obtain an aqueous phase.

Subsequently, the aqueous phase, and liquid hydrogenated coconut oil heated to 60°C were placed in a container, and then the resulting mixture was further warmed to 60°C. The mixture was stirred using a homomixer (T.K. Robomix, manufactured by PRIMIX Corporation) at 10,000 rpm for 1 minute, and then at 3000 rpm for 20 to 60 minutes, to obtain oil-in-water Pickering emulsion O.

### (Example 11)

The same operation as in Example 10 was carried out except that the amount of the sucrose fatty acid ester (S-1670) added was as shown in Table 10, to obtain oil-in-water Pickering emulsion P.

### (Example 12)

The same operation as in Example 10 was carried out except that the amount of the sucrose fatty acid ester (S-1670) added was as shown in Table 10, to obtain oil-in-water Pickering emulsion Q.

### (Example 13)

The same operation as in Example 10 was carried out except that sucrose fatty acid ester (S-970) was used instead of sucrose fatty acid ester (S-1670), to obtain oil-in-water Pickering emulsion R.

### (Example 14)

The same operation as in Example 10 was carried out except that sucrose fatty acid ester (S-570) was used instead of sucrose fatty acid ester (S-1670), to obtain oil-in-water Pickering emulsion S.

### (Example 15)

The same operation as in Example 10 was carried out except that polyglycerin fatty acid ester (SWA10D) was used instead of sucrose fatty acid ester (S-1670), and that components were mixed as shown in Table 10, to obtain oil-in-water Pickering emulsion T.

### (Example 16)

The same operation as in Example 10 was carried out except that polyglycerin fatty acid ester (S-28D) was used instead of sucrose fatty acid ester (S-1670), to obtain oil-in-water Pickering emulsion U.

**Table 10**

| | Example 10 | Example 11 | Example 12 | Example 13 | Example 14 | Example 15 | Example 16 |
|---|---|---|---|---|---|---|---|
| Demineralized water (parts by weight) | Remainder | Remainder | Remainder | Remainder | Remainder | Remainder | Remainder |
| Crushed protein (parts by weight) | 1.75 | 1.75 | 1.75 | 1.75 | 1.75 | 1.75 | 1.75 |
| S-1670 (parts by weight) | 4.4×10⁻³ | 2.2×10⁻³ | 4.4×10⁻⁴ | | | | |
| S-970 (parts by weight) | | | | 4.4×10⁻³ | | | |
| S-570 (parts by weight) | | | | | 4.4×10⁻³ | | |
| Polyglycerin fatty acid ester contained in SWA10D (parts by weiaht) | | | | | | 4.4×10⁻³ | |
| S-28D (parts by weight) | | | | | | | 4.4×10⁻³ |
| Ampniphilic substances/solid particles (parts by weiaht) | 0.0025 | 0.00125 | 0.000250 | 0.00250 | 0.0025 | 0.00250 | 0.00250 |
| Hydrogenated coconut oil (parts by weight) | 30 | 30 | 30 | 30 | 30 | 30 | 30 |
| Total amount (parts by weight) | 100 | 100 | 100 | 100 | 100 | 100 | 100 |
| Oil phase/aqueous phase (parts by weight) | 30/70 | 30/70 | 30/70 | 30/70 | 30/70 | 30/70 | 30/70 |

### <Evaluation of Heat Stabilities of Oil-in-Water Emulsions>

Each of the Pickering emulsions according to Examples 10 to 16 was placed in a container, and heated under the conditions shown in the following table. Thereafter, the presence or absence of oil phase separation was visually observed from the side and top of the container. The evaluation results are shown in Table 11. The evaluation criteria are as follows.
Good: No oil phase separation was found by observation from the side and top.
Normal: No oil phase separation was found by observation from the side, but a small number of oil droplets were found by observation from the top.
Poor: Oil phase separation was found by observation from the side.

Each of the Pickering emulsions according to Examples 10 to 16 was placed in a container, and kept at 60°C. Thereafter, the container was immersed in a water bath for not less than 30 minutes, to thereby cool the container to 25°C. Thereafter, the container was inverted, and vibration was applied to the container. The fluidity and the presence or absence of aggregation of the Pickering emulsion after the cooling were visually observed. The evaluation results are shown in Table 11. The evaluation standard was as follows.
Good: No aggregates in the emulsion; good fluidity.
Poor: Generation of aggregates in the emulsion; poor fluidity.

**Table 11**

| | Resistance to continuous heating | | | | Cooling resistance |
|---|---|---|---|---|---|
| | 60°C | 65°C | 75°C | 95°C | 60°C→25°C |
| | 60 min | 30 min | 15 sec | 10 min | |
| Example 10 | Good | - | - | - | Good |
| Example 11 | Good | - | - | - | Good |
| Example 12 | Good | - | - | - | Good |
| Example 13 | Good | Good | Good | Normal | Good |
| Example 14 | Good | Good | - | Normal | Good |
| Example 15 | Good | Good | - | - | Good |
| Example 16 | Good | Good | - | Good | Good |

As is evident from the results, the Pickering emulsions prepared by adding a sucrose fatty acid ester or polyglycerin fatty acid ester, and rice-derived protein in combination, showed no oil separation even at high temperature, and were capable of maintaining a stable emulsion state. They also had good cooling resistance.

### <Preparation of Oil-in-Water Emulsions>

Oil-in-water emulsions were prepared using a product prepared by subjecting rice-derived protein (ORYZA POTEIN^{™}-P70, manufactured by Oryza Oil & Fat Chemical Co., Ltd; protein obtained from seeds of a gramineous plant *(Oryza sativa* Linne)) to crushing treatment for 4 hours using a paint shaker (PAINT SHAKER, manufactured by TOYOSEIKI) and 1-mm-diameter zirconia beads (hereinafter the product is referred to as crushed protein), as solid particles; a sucrose fatty acid ester (RYOTO Sugar Ester S-1670, manufactured by Mitsubishi-Chemical Foods Corporation) as a nonionic amphiphilic substance; hydrogenated coconut oil as an oil phase component; and demineralized water as an aqueous phase component; such that the total amount was 100 parts by weight. Table 12 shows the amount of each component added (parts by weight) in each of Examples and Comparative Examples.

### (Example 17)

First, a sucrose fatty acid ester (S-1670) was dissolved in demineralized water with warming. To the resulting solution, rice-derived protein was added, and the resulting mixture was mixed to obtain a rice-derived protein dispersion. The rice-derived protein dispersion was prepared such that the ratio of the rice-derived protein was 10% by weight, and such that the ratio of the sucrose fatty acid ester added with respect to 1 part by weight of the rice-derived protein was 0.0025 parts by weight.

To 15 parts by weight of the thus prepared rice-derived dispersion, 20 parts by weight of 1-mm-diameter zirconia beads were added, and crushing treatment was carried out using a paint shaker (PAINT SHAKER, manufactured by TOYOSEIKI) for 4 hours, to obtain a crushed protein dispersion. The crushed protein dispersion and demineralized water were placed in a container, and the resulting mixture was mixed to obtain an aqueous phase.

Subsequently, the aqueous phase, and liquid hydrogenated coconut oil heated to 60°C were placed in a container, and then resulting mixture was further warmed to 60°C. The mixture was stirred using a homomixer (T.K. Robomix, manufactured by PRIMIX Corporation) at 10,000 rpm for 1 minute, and then at 3000 rpm for 20 minutes, to obtain oil-in-water Pickering emulsion V.

### (Example 18)

The same operation as in Example 17 was carried out except that the length of time of crushing using a paint shaker was 7 hours, and that the amount of the rice-derived protein added was as shown in Table 12, to obtain oil-in-water Pickering emulsion W.

### (Example 19)

The same operation as in Example 17 was carried out except that the amounts of the rice-derived protein, the preliminary sucrose fatty acid ester (S-570), and the hydrogenated coconut oil added were as shown in Table 12, to obtain oil-in-water Pickering emulsion X.

### (Example 20)

The same operation as in Example 19 was carried out except that a homogenizer (IKA T25 digital ULTRA TURRAX, shaft generator: S25N-10G) was used instead of the homomixer, to obtain oil-in-water Pickering emulsion Y.

**Table 12**

| | Example 17 | Example 18 | Example 19 | Example 20 |
|---|---|---|---|---|
| Demineralized water (parts by weight) | Remainder | Remainder | Remainder | Remainder |
| Crushed protein (parts by weight) | 4.90 | 5.60 | 1.75 | 1.75 |
| S-1670 (parts by weight) | 1.23×10⁻² | 1.40×10⁻² | | |
| S-570 (parts by weight) | | | 4.38×10⁻³ | 4.38×10⁻³ |
| Amphiphilic substances/solid particles (parts by weight) | 0.0025 | 0.0025 | 0.0025 | 0.0025 |
| Hydrogenated coconut oil (parts by weight) | 30 | 30 | 30 | 30 |
| Total amount (parts by weight) | 100 | 100 | 100 | 100 |

### <Evaluation of Heat Stabilities of Oil-in-Water Emulsions>

Each of the Pickering emulsions according to Examples 17 and 18 was placed in a container, and heated by the thermal history under the conditions shown below in Table 13, followed by performing evaluation of heat resistance. Thereafter, the presence or absence of oil phase separation was visually observed from the side and top of the container. The evaluation results are shown in Table 13. The evaluation criteria are as follows. Good: No oil phase separation was found by observation from the side and top.
Normal: No oil phase separation was found by observation from the side, but a small number of oil droplets were found by observation from the top.
Poor: Oil phase separation was found by observation from the side.

Each of the Pickering emulsions according to Examples 17 and 18 was placed in a container, and the container was immersed in a water bath for not less than 30 minutes, to thereby cool the container to 25°C or 4°C. Thereafter, the container was inverted, and vibration was applied to the container. The fluidity and the presence or absence of aggregation of the Pickering emulsion after the cooling were visually observed. The evaluation results are shown in the "cooling resistance" columns of Table 13. The evaluation criteria are as follows.
Good: No aggregates in the emulsion; good fluidity.
Poor: Generation of aggregates in the emulsion; poor fluidity.

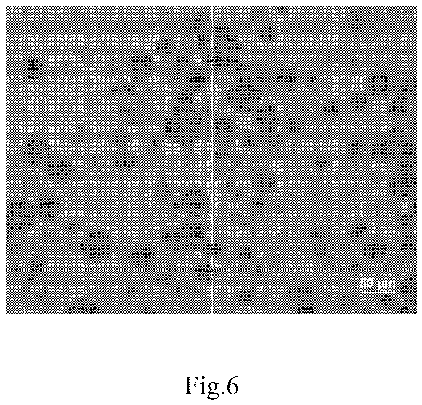

Fig. 6 shows a micrograph of the Pickering emulsion according to Example 18 at 60°C. The observation was carried out using a liquid prepared by 10-fold dilution of the Pickering emulsion according to Example 18 with water warmed to 60°C. As a result of measurement of 15 arbitrary oil droplets found in an image area of 464 × 623 µm, the oil droplet size was 45 µm ± 8.0 µm.

**Table 13**

| | Resistance to continuous heating | Cooling resistance 1 | Cooling resistance 2 | Heat resistance after cooling 1 | Heat resistance after cooling 2 | Heat resistance after cooling 3 |
|---|---|---|---|---|---|---|
| | 60°C 60 min | 60°C →25°C | 60°C →4°C | 60°C →4°C →65°C 30 min | 60°C →4°C →75°C 15 sec | 60°C →4°C →95°C 10 sec |
| Example 17 | Good | Good | Good | Good | Normal | Normal |
| Example 18 | Good | Good | Good | Normal | Normal | Normal |

### (Evaluation of Amount of Solid Particles Adsorbed)

The Pickering emulsion X according to Example 19 and the Pickering emulsion Y according to Example 20 were observed by microscopy using a polarization microscope ECLIPSE LV100N POL, manufactured by Nikon Corporation, and image integration software NIS-Elements Ver 3.2, manufactured by Nikon Corporation. The observation was carried out using a slide glass warmed to 60°C. Each sample was preliminarily diluted 10-fold with demineralized water warmed to 60°C. As a result of the microscopy, emulsion formation was confirmed. The amount of solid particles adsorbed was found to be larger in Pickering emulsion X, which was prepared by emulsification using a homomixer, than in Pickering emulsion Y, which was prepared by emulsification using a homogenizer.

## Claims

1. An oil-in-water Pickering emulsion comprising a solid particle, an oil phase component, an aqueous phase component, and an amphiphilic substance, wherein the solid particle is a rice-derived protein.

2. The oil-in-water Pickering emulsion according to claim 1, wherein the content of the amphiphilic substance is not less than 0.001% by weight and not more than 0.01% by weight, with respect to the total amount of the Pickering emulsion.

3. The oil-in-water Pickering emulsion according to claim 1 or 2, wherein the amphiphilic substance is a nonionic amphiphilic substance.

4. The oil-in-water Pickering emulsion according to claim 3, wherein the nonionic amphiphilic substance has an HLB of not less than 3.

5. The oil-in-water Pickering emulsion according to claim 3 or 4, wherein the nonionic amphiphilic substance is a sucrose fatty acid ester and/or polyglycerin fatty acid ester.

6. The oil-in-water Pickering emulsion according to claim 5, wherein the monoester content in the sucrose fatty acid ester is 20 to 100% by weight.

7. The oil-in-water Pickering emulsion according to any one of claims 1 to 6, wherein the oil phase has an average particle size of not less than 2.2 µm, measured as described in the specification using a laser diffraction/scattering particle
size distribution measuring apparatus.

8. The oil-in-water Pickering emulsion according to any one of claims 1 to 7, wherein the oil phase has an average particle size of not less than 5 µm, measured as described in the specification using a laser diffraction/scattering particle
size distribution measuring apparatus.

9. The oil-in-water Pickering emulsion according to any one of claims 1 to 8, wherein the weight of the amphiphilic substance with respect to the weight of the solid particle is not less than 0.0025 and not more than 0.005.

10. The oil-in-water Pickering emulsion according to any one of claims 1 to 9, wherein the molecular weight of the amphiphilic substance is not more than 5000.

11. A food comprising the oil-in-water Pickering emulsion according to any one of claims 1 to 10.

12. A milk substitute comprising the oil-in-water Pickering emulsion according to any one of claims 1 to 10.

13. A pharmaceutical product comprising the oil-in-water Pickering emulsion according to any one of claims 1 to 10.

14. A cosmetic comprising the oil-in-water Pickering emulsion according to any one of claims 1 to 10.

15. A personal care product comprising the oil-in-water Pickering emulsion according to any one of claims 1 to 10.

## Patentansprüche

1. Öl-in-Wasser-Beizemulsion umfassend ein festes Partikel, eine Ölphasenkomponente, eine wässrige Phasenkomponente, und eine amphiphile Substanz, wobei das feste Partikel ein aus Reis gewonnenes Protein ist.

2. Öl-in-Wasser-Beizemulsion gemäß Anspruch 1, wobei der Gehalt der amphiphilen Substanz nicht weniger als 0,001 Gew.-% und nicht mehr als 0,01 Gew.-%, bezogen auf die Gesamtmenge der Beizemulsion, beträgt.

3. Öl-in-Wasser-Beizemulsion gemäß Anspruch 1 oder 2, wobei die amphiphile Substanz eine nichtionische amphiphile Substanz ist.

4. Öl-in-Wasser-Beizemulsion gemäß Anspruch 3, wobei die nichtionische amphiphile Substanz einen HLB-Wert von nicht weniger als 3 aufweist.

5. Öl-in-Wasser-Beizemulsion gemäß Anspruch 3 oder 4, wobei die nichtionische amphiphile Substanz ein Saccharosefettsäureester und/oder Polyglycerinfettsäureester ist.

6. Öl-in-Wasser-Beizemulsion gemäß Anspruch 5, wobei der Monoestergehalt im Saccharosefettsäureester 20 bis 100 Gew.-% beträgt.

7. Öl-in-Wasser-Beizemulsion gemäß mindestens einem der Ansprüche 1 bis 6, wobei die Ölphase eine durchschnittliche Partikelgröße von nicht weniger als 2,2 µm aufweist, gemessen wie in der Beschreibung beschrieben unter Verwendung eines Laserbeugungs- /Streuungs-Partikelgrößenverteilungsmessgeräts.

8. Öl-in-Wasser-Beizemulsion gemäß mindestens einem der Ansprüche 1 bis 7, wobei die Ölphase eine durchschnittliche Partikelgröße von nicht weniger als 5 µm aufweist, gemessen wie in der Beschreibung beschrieben unter Verwendung eines Laserbeugungs- /Streuungs-Partikelgrößenverteilungsmessgeräts.

9. Öl-in-Wasser-Beizemulsion gemäß mindestens einem der Ansprüche 1 bis 8, wobei das Gewicht der amphiphilen Substanz im Verhältnis zum Gewicht des festen Partikels nicht weniger als 0,0025 und nicht mehr als 0,005 beträgt.

10. Öl-in-Wasser-Beizemulsion gemäß mindestens einem der Ansprüche 1 bis 9, wobei das Molekulargewicht der amphiphilen Substanz nicht mehr als 5000 beträgt.

11. Lebensmittel umfassend die Öl-in-Wasser-Beizemulsion gemäß mindestens einem der Ansprüche 1 bis 10.

12. Milchersatzprodukt umfassend die Öl-in-Wasser-Beizemulsion gemäß mindestens einem der Ansprüche 1 bis 10.

13. Pharmazeutisches Produkt, umfassend die Öl-in-Wasser-Beizemulsion gemäß mindestens einem der Ansprüche 1 bis 10.

14. Kosmetikum, umfassend die Öl-in-Wasser-Beizemulsion gemäß mindestens einem der Ansprüche 1 bis 10.

15. Körperpflegeprodukt, umfassend die Öl-in-Wasser-Beizemulsion gemäß mindestens einem der Ansprüche 1 bis 10.

## Revendications

1. Une émulsion Pickering huile dans eau comprenant une particule solide, un composant de phase huileuse, un composant de phase aqueuse et une substance amphiphile, dans laquelle la particule solide est une protéine dérivée du riz.

2. L'émulsion Pickering de type huile dans l'eau selon la revendication 1, dans laquelle la teneur en substance amphiphile n'est pas inférieure à 0,001 % en poids et n'est pas supérieure à 0,01 % en poids, par rapport à la quantité totale de l'émulsion de Pickering.

3. L'émulsion Pickering huile dans l'eau selon la revendication 1 ou 2, dans laquelle la substance amphiphile est une substance amphiphile non ionique.

4. L'émulsion Pickering huile dans l'eau selon la revendication 3, dans laquelle la substance amphiphile non ionique a un HLB d'au moins 3.

5. L'émulsion Pickering de type huile dans l'eau selon la revendication 3 ou 4, dans laquelle la substance amphiphile non ionique est un ester d'acide gras de saccharose et/ou un ester d'acide gras de polyglycérine.

6. L'émulsion Pickering de type huile dans l'eau selon la revendication 5, dans laquelle la teneur en monoester dans l'ester d'acide gras de saccharose est de 20 à 100 % en poids.

7. L'émulsion Pickering de type huile dans l'eau selon l'une quelconque des revendications 1 à 6, dans laquelle la phase huileuse a une taille moyenne de particules non inférieure à 2,2 µm, mesurée comme décrit dans la description à l'aide d'un appareil de mesure de la distribution granulométrique par diffraction/diffusion laser

8. L'émulsion Pickering de type huile dans l'eau selon l'une quelconque des revendications 1 à 7, dans laquelle la phase huileuse a une taille moyenne de particules non inférieure à 5 µm, mesurée comme décrit dans la description à l'aide d'un appareil de mesure de la distribution granulométrique par diffraction/diffusion laser

9. L'émulsion Pickering de type huile dans l'eau selon l'une quelconque des revendications 1 à 8, dans laquelle le poids de la substance amphiphile par rapport au poids de la particule solide n'est pas inférieur à 0,0025 et n'est pas supérieur à 0,005.

10. L'émulsion Pickering de type huile dans l'eau selon l'une quelconque des revendications 1 à 9, dans laquelle le poids moléculaire de la substance amphiphile n'est pas supérieur à 5000.

11. Un aliment comprenant l'émulsion Pickering de type huile dans l'eau selon l'une quelconque des revendications 1 à 10.

12. Un substitut de lait comprenant l'émulsion de Pickering de type huile dans l'eau selon l'une quelconque des revendications 1 à 10.

13. Un produit pharmaceutique comprenant l'émulsion de Pickering de type huile dans l'eau selon l'une quelconque des revendications 1 à 10.

14. Un produit cosmétique comprenant l'émulsion de Pickering de type huile dans l'eau selon l'une quelconque des revendications 1 à 10.

15. Un produit de soins personnels comprenant l'émulsion de Pickering de type huile dans l'eau selon l'une quelconque des revendications 1 à 10.
